# EUROPÄISCHE PATENTSCHRIFT

(11) **EP 1 648 540 B1**
(45) Veröffentlichungstag und Bekanntmachung des Hinweises auf die Patenterteilung: **18.09.2013**
(21) Anmeldenummer: 04762497.8
(22) Anmeldetag: 23.07.2004
(51) Int. Cl.: A61M 5/32, A61M 5/315, A61M 5/20

(54) **INJEKTIONSVORRICHTUNG**
INJECTION DEVICE
DISPOSITIF D'INJECTION

(30) Priorität: 01.08.2003 DE 20311996 U
(43) Veröffentlichungstag der Anmeldung: 26.04.2006
(73) Patentinhaber: Bayer Intellectual Property GmbH, 40789 Monheim (DE)
(72) Erfinder: WEBER, Wilfried, 72296 Schopfloch (DE)
(74) Vertreter: Schön, Thilo
(86) Internationale Anmeldenummer: PCT/DE2004/001649
(87) Internationale Veröffentlichungsnummer: WO 2005/011780

(56) Entgegenhaltungen:
- DE-C- 356 704
- FR-A- 2 519 866

## Beschreibung

### Technischer Hintergrund

Es sind eine Vielzahl von Injektionsvorrichtungen bekannt, mit deren Hilfe eine eingelegte Spritze so positioniert werden kann, dass ein problemloses Einstechen in die Haut bis zur erforderlichen Tiefe und die Injektion des Medikaments vorgenommen werden kann, ohne die Spritze unmittelbar mit der Hand zu betätigen. In jedem Fall hat eine solche Injektionsvorrichtung zum Zweck, die Sicherheit der Injektion und den Komfort bei der Handhabung zu verbessern, um allen Patienten auch ohne permanente sachgemäße Anleitung die oft täglich oder auch mehrfach täglich erforderlichen Injektionen selbständig zu ermöglichen, was auch eine erhebliche Kostenersparnis darstellt.

### Stand der Technik

Es sind Injektionsvorrichtungen bekannt, bei denen zur Erhöhung des Komforts und der Sicherheit eine automatische Aufeinanderfolge des Einstechens der Nadel der Spritze und eine anschließende Injektion durchgerührt wird, z.B. aus der EP 1 233 801.

Die FR 2 519 866 A betrifft einen Spritzenmechanismus, mit dem durch koaxial zueinander angeordnete Kanülen drei verschiedene Arzneimittel aus drei Kartuschen in verschiedene Einspritztiefen injiziert werden können. Ein Injektionsschlitten nimmt die drei Kartuschen auf und wird von einem Betätigungselement zur Injektion bewegt, wozu drei Kolben dienen, die von Federn beaufschlagt werden: Mittels einer ersten (äußeren) Feder wird bei Freigabe des Betätigungselements das Ensemble aus Betätigungselement mit den Kolben und dem Injektionsschlitten nach vorne geschoben, die beiden koaxialen Nadeln herausgedrückt und somit der Einstechhub bewirkt. Hierbei wird eine Feder gegen die Einstechrichtung gespannt. Danach werden weitere Federn aktiviert und die in den drei Kartuschen befindlichen Arzneimittel durch die beiden koaxialen Nadeln injiziert und somit der Injektionshub durchgeführt. Nach erfolgter Injektion kann diese Injektionsvorrichtung zur Entnahme der leeren Kartuschen geöffnet werden, indem ihre untere und obere Hälfte getrennt werden, dabei schiebt die Feder die Nadeln zu deren Schutz zurück.

Eine Offenbarung weiterer Funktionen dieses Spritzenmechanismus ist der Beschreibung zu diesem Dokument nicht zu entnehmen und auch aus den Figuren nicht ersichtlich.

Nach Beendigen der Injektion muss die Injektionsvorrichtung vom Patienten zum Herausziehen der Nadel von der Einstichstelle weg bewegt werden. Dies muss möglichst senkrecht zur Hautoberfläche und mit ruhiger Hand erfolgen, um Verletzungen durch die Nadel zu vermeiden. Dies ist bei den oben genannten Vorrichtungen nicht gewährleistet, vielmehr wird im Extremfall durch das gegenüber einer Spritze wesentlich größere Eigengewicht der Injektionsvorrichtung eine sichere Abnahme der Nadel sogar noch schwieriger.

Die DE 356 704 C zeigt eine konstruktive Möglichkeit, auch das Herausziehen der Nadel einer Injektionsvorrichtung mittels dreier teleskopartig angeordneter Röhren automatisch durchzuführen:
Der Einstechhub wird hierbei durch eine erste Feder bewirkt, die auf eine innere Röhre des Spritzenmechanismus wirkt, in der die Spritze gehalten ist. Nach dem Einstechhub wird eine zweite Feder freigegeben, die vorher durch Herausziehen einer Zahnstange als Betätigungselement vorgespannt wird. Die freigegebene zweite Feder bewirkt den Injektionshub, indem sie den Spritzenkolben beaufschlagt. Schließlich wird durch die Bewegung der Zahnstange am Ende des Injektionshubes eine dritte Feder freigegeben, die die Röhren sofort wieder auseinandertreibt und die Nadel aus der Einstichstelle wieder herauszieht.

Diese Patentschrift beschreibt somit eine Vorrichtung, die zwar eine gesteuerte Abfolge von Einstechhub, Injektionshub und Rückholhub durchführt, deren konstruktive Lösung aber darauf basiert, dass für jeden Hub separate Führungs- und Federelemente vorgesehen sind, die sukzessiv ausgelöst werden.

Bei dieser Vorrichtung wird somit jeder der drei Hübe durch eine konstruktiv weitgehend autarke Bauteilgruppe ausgeführt, mit der entscheidenden Folge, dass auch der Patient drei Handgriffe zum Spannen der drei Federn vornehmen muss, nämlich durch Herausziehen der inneren Röhre (Spannung der Einstechfeder), durch Herausziehen der Zahnstange (Spannung der Injektionsfeder) und durch Zusammenschieben der beiden äußeren Röhren nach dem Ende eines Zyklus (Spannung der Rückholfeder).

Diese Spannung der drei Federn durch separate, zeitlich getrennte Handgriffe, die vor und nach dem (selbst automatisch ablaufenden) Injektionszyklus erhöhte Aufmerksamkeit seitens des Patienten erfordern, um durch zeitversetztes Spannen der drei Federn erneut die Betriebsbereitschaft für einen neuen Injektionszyklus herzustellen.

Durch den außen liegenden Zahnstangenmechanismus und die frei aussen angeordneten Steuerelemente, z.B. Sperrklinke 14 und Hebel 15, besteht auch die Gefahr einer unbeabsichtigten, unkoordinierten Aktivierung von einzelnen Hüben mit der Folge von Fehlfunktionen.

Diese vorbekannte Vorrichtung ist somit trotz des Automatisierungsgrades der Hübe nur schwerfällig zu handhaben und damit für den täglichen Gebrauch durch Laien nicht geeignet. Zusammen mit ihrem unhandlichen Aufbau genügt diese Injektionsvorrichtung daher nicht den heutigen Anforderungen an maximalem Komfort durch minimale Handhabungsanforderungen.

Die FR 2 616 221 A1 zeigt ebenfalls eine Injektionsvorrichtung mit einer Abfolge von Einstechhub, Injektionshub und Rückholhub, die im Gegensatz zur oben genannten DE 356 704 C durch eine einzige gerichtete lineare Bewegung eines Betätigungselementes in ihrer automatischen Aufeinanderfolge gesteuert werden.

Zur Halterung der Spritze dient bei dieser Injektionsvorrichtung wesentlich ein stationäres Führungsteil 20/181 für das vordere Ende der Spritze. Beim Ablauf des Einstechhubes wird eine speziell für den Rückholhub in diesem stationären Führungsteil eingelegte Feder 23/123 durch die Frontseite der Spritze vorgespannt, die dann den Rückholhub nach dem Ende des Injektionshubes bewirkt.

Das hülsenförmige, stationäre Führungsteil mit der eingelegten Rückholfeder hat jedoch den Nachteil, dass die Injektionsvorrichtung ausschließlich für ein bestimmtes Spritzenformat mit bestimmtem Außendurchmesser einsetzbar ist, wobei zudem auch eine relativ große Herstellungspräzision einzuhalten ist, damit einerseits eine sichere Führung der Spritze während des Einstechhubes gewährleistet ist, andererseits aber genügend Spiel vorhanden ist, um Verklemmungen oder Verkantungen und damit eine Fehlfunktion der Injektionsvorrichtung zu vermeiden.

Der Rückholweg während des Rückholhubes hängt bei den beiden vorgenannten Injektionsvorrichtungen ausschließlich von der Vorspannung der separaten Rückholfeder ab; dies bedeutet, dass zur Einhaltung eines definierten Rückholhubes, der in etwa dem gegenläufigen Einstechhub entsprechen soll, eine präzise Auswahl/Dimensionierung der Rückholfeder erforderlich ist, die wiederum mit den Reibungseigenschaften des Spritzenvorderteils im stationären Führungsteil zusammenhängt.

Die Konzeption des stationären Führungsteils mit der eingelegten Rückholfeder ist daher prinzipiell einfach, in ihrer konstruktiven Auslegung für die Praxis aber schwierig und birgt das Risiko von Fehlfunktionen.

### Darstellung der Erfindung

Es ist Aufgabe der Erfindung, die Handhabung der gattungsbildenden Injektionsvorrichtung einfacher und sicherer zu gestalten.

Diese Aufgabe wird gemäss den Merkmalen des Anspruchs 1 gelöst.

Die Erfindung schafft somit eine Injektionsvorrichtung, die mittels einer einzigen gerichteten linearen Bewegung das Einstechen der Nadel in eine definierte Tiefe, die Injektion des Arzneimittels, und nach vollständiger Injektion einen Rückholhub erzeugt, der ein Zurückziehen der Nadel in das Gehäuse und damit aus der Einstichstelle bewirkt. Die Antriebskraft für die lineare Bewegung kann manuell entweder unmittelbar oder unter Zwischenschaltung von Kraftspeichern erzeugt werden. Zu einer definierten Rückholbewegung der Spritze wird ein zusätzlicher Schlitten eingesetzt, der funktional zwischen Betätigungselement und Injektionsschlitten angeordnet ist.

Der Unterschied zur technischen Lehre der DE 356 704 C ist somit in der Auswahl und Zuordnung der Bauteile zu sehen, die es insbesondere ermöglichen, dass der Patient mit nur einem Handgriff die gesamte Betätigungsarbeit für den gesamten Ablauf leistet, die Injektionsvorrichtung bildet somit eine "integrierte" Baueinheit.

Der Unterschied zur technischen Lehre der gattungsbildenden FR-A-2 616 221 und der DE 356 704 C ist darin zu sehen, dass die Führung der Spritze beim Rückholhub durch ein Schlitten-Bauteil erfolgt, das keiner Abstimmung auf den Spritzendurchmesser bedarf. Als Führungseinrichtung dient ausschließlich eine Spritzenaufnahme, die Teil des Injektionsschlittens ist und die eine exakte Steuerung des Wegs des Rückholhubs gewährleistet, ohne Wechselwirkung einer in einer zylindrischen Führungseinrichtung gehaltenen, auf die Spritzenfront wirkenden Rückholfeder, mit deren mechanischen und dynamischen Unwägbarkeiten beim Rückholhub.

Am Ende des Rückholhubs kann ein Signalton erzeugt werden. Nach diesem Signalton kann der Patient die gesamte Injektionsvorrichtung ohne besondere Vorsicht oder Sorgfalt von der Injektionsstelle abheben, da die Nadel aus der Einstichstelle zurückgezogen ist.

Die Nadel steht nach abgeschlossener Injektion nicht aus der Injektionsvorrichtung heraus, deshalb besteht auch keine Verletzungsgefahr mehr bei der Handhabung der Injektionsvorrichtung nach der Injektion.

Wenn nach dem Einlegen der Spritze die Schutzkappe abgezogen wird und nach dem Injektionsvorgang, vor der Entnahme der Spritze die Schutzkappe wieder aufgeschoben wird, sieht der Patient weder vor noch nach der Injektion bei einer vorgefüllten Spritze die Nadel nicht, was die Handhabung der Injektionsvorrichtung insbesondere für solche Patienten erleichtert, die unter einer sogenannten "Nadelphobie" leiden.

Vorteilhafte Weiterbildungen der erfindungsgemäßen Injektionsvorrichtung sind den Unteransprüchen zu entnehmen.

### Kurze Beschreibung der Zeichnungen

Mehrere Ausführungsbeispiele der erfindungsgemäßen Injektionsvorrichtung werden nun anhand von Figuren näher erläutert. Es zeigen:
- Fig. 1:: ein erstes Ausführungsbeispiel in einem ersten Längsschnitt in der Ebene X-X der Figur 2 in der Bereitschaftsposition mit eingelegter Spritze,
- Fig. 2:: das erste Ausführungsbeispiel in der Aufsicht ohne Spritze,
- Fig. 3:: einen Schnitt in der Ebene A-A der Figur 1,
- Fig. 4:: einen Teilschnitt in der Ebene B-B der Figur 1,
- Fig. 5:: einen Schnitt in der Ebene C-C der Figur 2,
- Fig. 6:: einen zweiten Längsschnitt in der Injektionsposition nach abgeschlossenem Einstechhub und Injektionshub,
- Fig. 7:: einen dritten Längsschnitt nach Beendigung des Rückhubs
- Fig. 8:: einen Schnitt entsprechend Figur 3 durch eine erste Variante des ersten Ausführungsbeispiels mit einem Getriebe,
- Fig. 9:: einen Teilschnitt entsprechend Figur 1 durch eine zweite Variante des ersten Ausführungsbeispiels,
- Fig. 10:: einen Teilschnitt entsprechend Figur 1 in der Ebene F-F der Figur 12 durch eine dritte Variante des ersten Ausführungsbeispiels,
- Fig. 11:: einen Schnitt in der Ebene G-G der Figur 10,
- Fig. 12:: einen Schnitt in der Ebene E-E der Figur 10,
- Fig. 13:: einen Teilschnitt entsprechend Figur 10 durch eine vierte Variante des ersten Ausführungsbeispiels,
- Fig. 14:: ein zweites Ausführungsbeispiel in einem ersten Längsschnitt in der Ebene H-H der Figur 15 in der Bereitschaftsposition mit eingelegter Spritze,
- Fig. 15:: das zweite Ausführungsbeispiel in einer ersten Aufsicht ohne Spritze,
- Fig. 16:: einen zweiten Längsschnitt in der Ebene H-H der Figur 17, nach dem Einstechhub während des Injektionshubs,
- Fig. 17:: eine zweite Aufsicht gemäß Figur 15 (ohne Spritze) während des Injektionshubs,
- Fig. 18:: einen Schnitt in der Ebene K-K der Figur 15,
- Fig. 19:: einen Schnitt in der Ebene L-L der Figur 15,
- Fig. 20:: einen Schnitt in der Ebene M-M der Figur 15,
- Fig. 21:: ein drittes Ausführungsbeispiel in einem Längsschnitt mit eingelegter Spritze,
- Fig. 22:: eine Gesamtansicht der Injektionsvorrichtung gemäß Figur 21,
- Fig. 23:: eine perspektivische Ansicht der beiden Hälften des Aufnahmerahmens
- Fig. 24:: eine erste perspektivische Ansicht der Spritzenaufnahme und Stössel
- Fig. 25:: eine zweite perspektivische Ansicht der Spritzenaufnahme und Stössel
- Fig. 26:: eine erste perspektivische Ansicht des Vorschubschlittens
- Fig. 27:: eine zweite perspektivische Ansicht des Vorschubschlittens
- Fig. 28:: eine perspektivische Ansicht des Zahnradschlittens
- Fig. 29:: eine perspektivische Ansicht der Ladezugstange mit Rollfeder
- Fig. 30:: eine erste perspektivische Ansicht des Lademechanismus,
- Fig. 31:: eine zweite perspektivische Ansicht des Lademechanismus,
- Fig. 32:: eine perspektivische Ansicht des Klingelmechanismus
- Fig. 33:: eine perspektivische Ansicht der Oberseite der Injektionsvorrichtung gemäß Figur 22 mit beiden Hälften des Aufnahmerahmens,
- Fig. 34:: eine perspektivische Ansicht der Oberseite mit einer Hälfte des Aufnahmerahmens gemäß Figur 22,
- Fig. 35:: eine perspektivische Ansicht der Unterseite mit einer Hälfte des Aufnahmerahmens gemäß Figur 24
- Fig. 36:: eine perspektivische Darstellung wesentlicher Funktionsbauteile in der Startposition mit eingelegter Spritze,
- Fig. 36A: einen ersten Längsschnitt durch die Funktionsbauteile gemäß Figur 36,
- Fig. 36B: einen zweiten Längsschnitt durch die Funktionsbauteile gemäß Figur 36,
- Fig. 36C: einen dritten Längsschnitt durch die Funktionsbauteile gemäß Figur 36,
- Fig. 37: eine perspektivische Teildarstellung wesentlicher Funktionsbauteile während des Einstechhubs,
- Fig. 37A: einen ersten Längsschnitt durch die Funktionsbauteile in ihrer Position gemäß Figur 37,
- Fig. 37B: einen zweiten Längsschnitt durch die Funktionsbauteile in ihrer Position gemäß Figur 37,
- Fig. 37C: einen dritten Längsschnitt durch die Funktionsbauteile in ihrer Position gemäß Figur 37,
- Fig. 38: eine perspektivische Teildarstellung wesentlicher Funktionsbauteile während des Einstechhubs,
- Fig. 38A: einen ersten Längsschnitt durch die Funktionsbauteile in ihrer Position gemäß Figur 38,
- Fig. 38B: einen zweiten Längsschnitt durch die Funktionsbauteile in ihrer Position gemäß Figur 38,
- Fig. 38C: einen dritten Längsschnitt durch die Funktionsbauteile in ihrer Postition gemäß Figur 38,
- Fig. 39: eine perspektivische Teildarstellung wesentlicher Funktionsbauteile nach Beendigung des Injektionshubs,
- Fig. 40: eine perspektivische Teildarstellung wesentlicher Funktionsbauteile vor Beginn des Rückholhubs,
- Fig. 41: eine perspektivische Teildarstellung wesentlicher Funktionsbauteile während des Rückholhubs,
- Fig. 41A: einen ersten Längsschnitt durch die Funktionsbauteile in ihrer Position gemäß Figur 41,
- Fig. 41B: einen zweiten Längsschnitt durch die Funktionsbauteile in ihrer Position gemäß Figur 41,
- Fig. 41C: einen dritten Längsschnitt durch die Funktionsbauteile in ihrer Position gemäß Figur 41,
- Fig. 42: eine perspektivische Teildarstellung wesentlicher Funktionsbauteile nach Beendigung des Rückholhubs,
- Fig. 42A: einen ersten Längsschnitt durch die Funktionsbauteile in ihrer Position gemäß Figur 42,
- Fig. 42B: einen zweiten Längsschnitt durch die Funktionsbauteile in ihrer Position gemäß Figur 42,
- Fig. 43: eine erste perspektivische Teildarstellung wesentlicher Funktionsbauteile während des Ladevorgangs,
- Fig. 44: eine zweite perspektivische Teildarstellung wesentlicher Funktionsbauteile während des Ladevorgangs,
- Fig. 44A: einen ersten Längsschnitt durch die Funktionsbauteile in ihrer Position gemäß Figur 43/44,
- Fig.44B: einen zweiten Längsschnitt durch die Funktionsbauteile in ihrer Position gemäß Figur 43/44,
- Fig. 44C: einen dritten Längsschnitt durch die Funktionsbauteile in ihrer Position gemäß Figur 43/44,
- Fig. 45: eine dritte perspektivische Teildarstellung wesentlicher Funktionsbauteile während des Ladevorgangs,
- Fig. 46: eine vierte perspektivische Teildarstellung wesentlicher Funktionsbauteile während des Ladevorgangs,
- Fig. 47: eine perspektivische Teildarstellung wesentlicher Funktionsbauteile nach dem Ladevorgang und nach dem Spritzenauswurf,
- Fig. 47A: einen ersten Längsschnitt durch die Funktionsbauteile in ihrer Position gemäß Figur 47,
- Fig.47B: einen zweiten Längsschnitt durch die Funktionsbauteile in ihrer Position gemäß Figur 47,
- Fig. 48A: ein viertes Ausführungsbeispiel in einem Längsschnitt mit eingelegter Spritze und einem Volumenadapter im Ausgangszustand,
- Fig. 48B: einen Schnitt nach Beendigung des Einstechhubs,
- Fig. 48C: einen Teilschnitt nach Beendigung des Injektionshubs,
- Fig. 49: eine perspektivische Teilansicht des Injektionsschlittens mit einem eingesetzten Volumenadapter,
- Fig. 50: einen Teilschnitt durch das vierte Ausführungsbeispiel mit größerem Volumenadapter,
- Fig. 51A: einen Teilschnitt einer ersten Variante der Antriebskopplung des vierten Ausführungsbeispiels im Ausgangszustand,
- Fig. 51B: einen Teilschnitt der ersten Variante nach Beendigung des Einstechhubs,
- Fig. 52: einen Teilschnitt durch eine zweite Variante der Antriebskopplung,
- Fig. 53: einen Teilschnitt durch eine dritte Variante der Antriebskopplung.

### Beschreibung der Ausführungsbeispiele

Im folgenden werden vier Ausführungsbeispiele beschrieben; der grundsätzliche Aufbau der Injektionsvorrichtung ist bei allen Ausführungsbeispielen wie folgt:
Die Spritze mit Kolben, Kolbenstange und Kanüle mit Nadel wird in eine Spritzenaufnahme eingelegt, mit deren Hilfe der Einstechhub H1 bewirkt wird, d. h., das Einstechen der Nadel in die Injektionsstelle. Hierzu ist die Spritzenaufnahme in einem Gehäuse axial verschiebbar gelagert. Zur Betätigung der Spritze nach dem Einstechhub, d. h., zur Injektion des Arzneimittels, dient ein Stössel, der relativ zur Spritzenaufnahme verschiebbar gehalten ist und der den Kolben der Spritze beaufschlagt (Injektionshub H2). Spritzenaufnahme und Stössel sind derart lösbar miteinander gekoppelt, dass nach dem Einstechhub H1 unmittelbar der Injektionshub H2 beginnt, d. h., während des Einstechhubs H1 sind Spritzenaufnahme und Stössel starr miteinander verbunden und bewegen sich gemeinsam im Gehäuse nach vorne, während des Injektionshubs H2 ist die Kopplung gelöst, die Spritzenaufnahme bleibt im Gehäuse stehen und nur der Stössel bewegt sich weiter nach vom.

Spritzenaufnahme und Stössel bilden zusammen den Injektionsschlitten.

Nach Beendigung der Injektion wird erfindungsgemäß der Injektionsschlitten wieder in seine Ausgangslage zurückgezogen (Rückholhub H3) und die Nadel wird aus der Haut vollständig herausgezogen.

Um diesen Ablauf (Einstechhub H1, Injektionshub H2, Rückholhub H3) zu steuern, ist ein Betätigungselement vorgesehen, das seinerseits aus mehreren Bauteilen besteht, und das dazu dient eine vom Patienten aufzubringende Aktion positions- und richtungsdefiniert in die Bewegungen des Injektionsschlittens umzusetzen. Die im Betätigungselement enthaltenen Bauteile sind z. B. Zahnräder, Schubstangen, Federn und ähnliche Elemente, die der unmittelbaren oder gespeicherten Bewegungskopplung und -erzeugung dienen.

Die vier Ausführungsbeispiele unterscheiden sich funktionell im wesentlichen in der Art der Aufbringung der erforderlichen Betätigungsarbeit durch den Patienten und deren Umsetzung in Einstechhub H1, Injektionshub H2 und Rückholhub H3. Dementsprechend sind unterschiedliche Rast- und Koppelelemente (Nasen, Zungen, Ausnehmungen, Anschläge etc.) positioniert, um die Arbeitsabläufe ineinander überzuleiten, abhängig von der Position der beweglichen Bauteile zueinander.

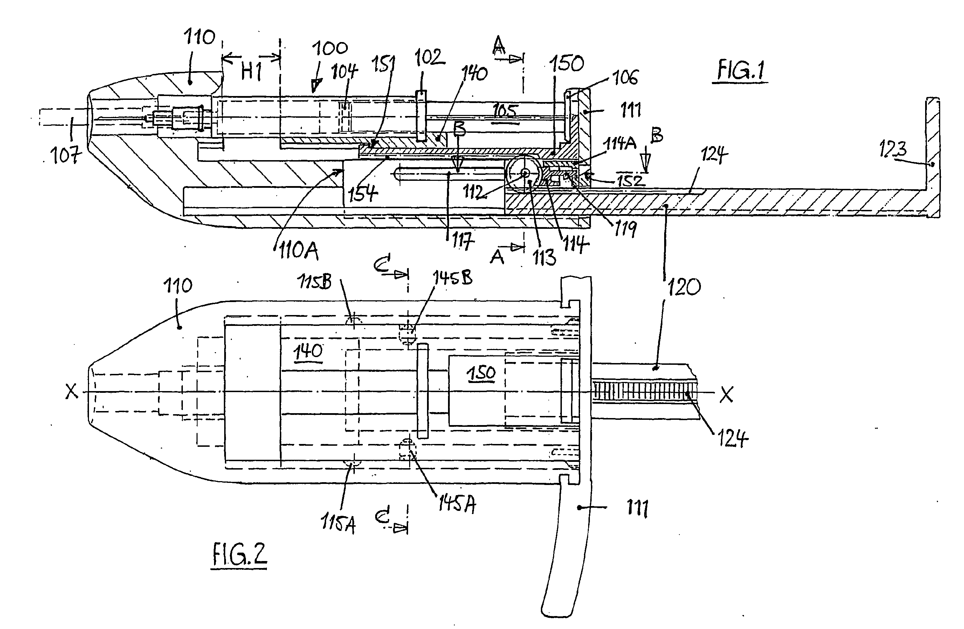

Beim ersten Ausführungsbeispiel (Figuren 1-13) erfolgt die Umsetzung unmittelbar, d. h., das Betätigungselement besteht im wesentlichen aus einer Schubstange, deren kontinuierliches Einschieben in das Gehäuse durch den Patienten nacheinander die Bewegungen des Injektionsschlittens hervorruft, wobei beim Übergang von Injektionshub H2 zum Rückholhub H3 die Bewegung der Schubstange und des Injektionsschlittens gegenläufig sind.

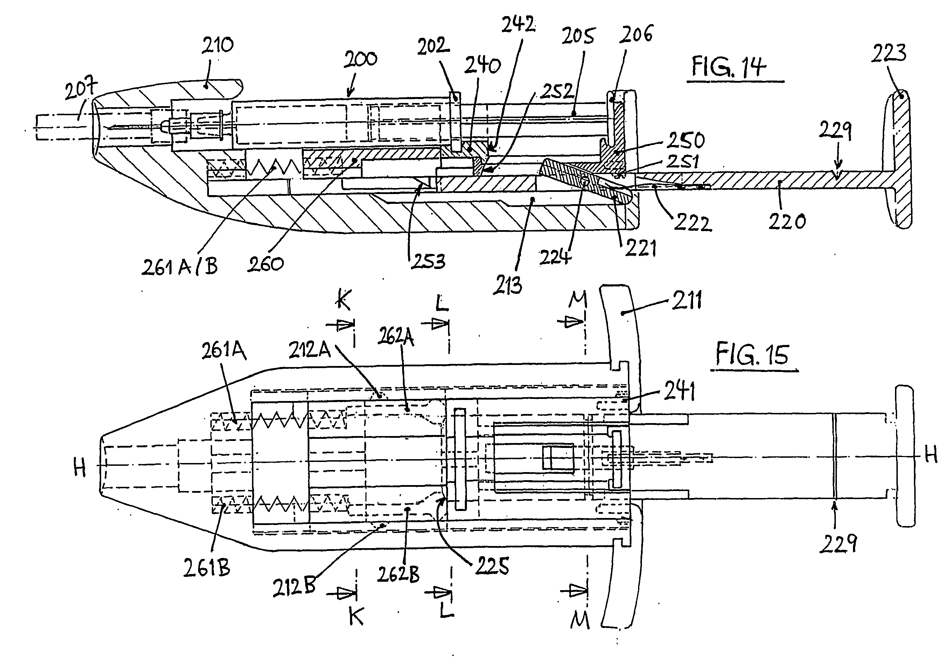

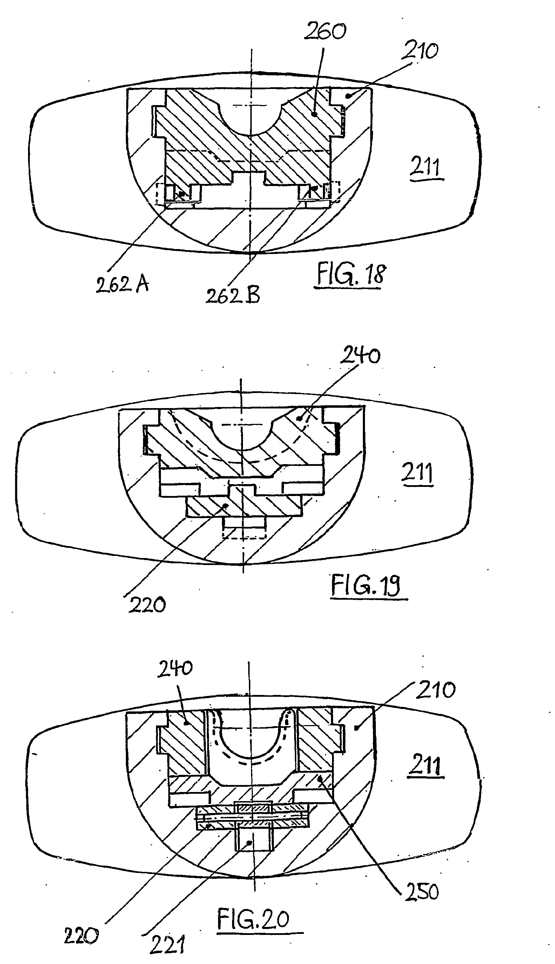

Beim zweiten Ausführungsbeispiel (Figuren 14-20) wird die Bewegung des Betätigungselements (Schubstange) vor Betätigung des Injektionsschlittens dazu verwendet, einen Federspeicher zu laden, der die für den Rückholhub H3 erforderliche Arbeit zum Rückführen des Injektionsschlittens bereitstellt. Die schlagartige Auslösung des Rückholhubs H3 durch Freisetzen der Federenergie hat den Vorteil einer impulsartigen Rückziehung der Nadel aus der Haut und minimiert daher weiter die oben beschriebenen Nachteile der bekannten Injektionsvorrichtungen.

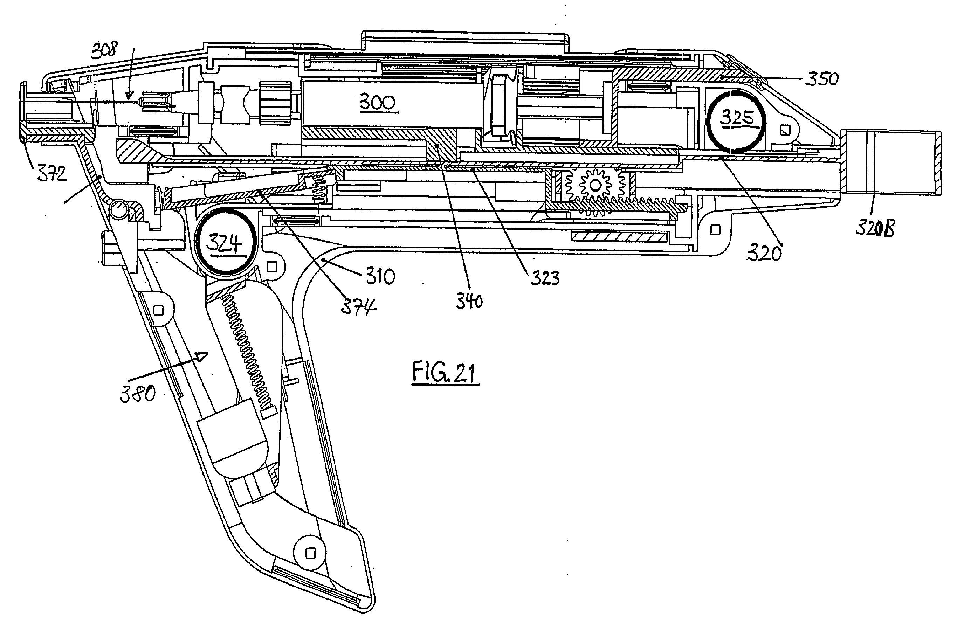

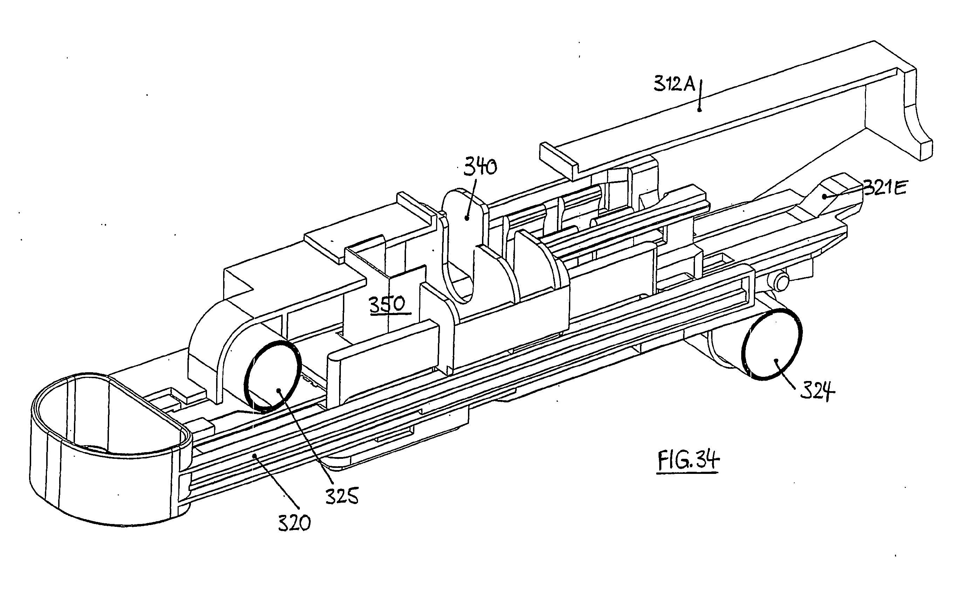

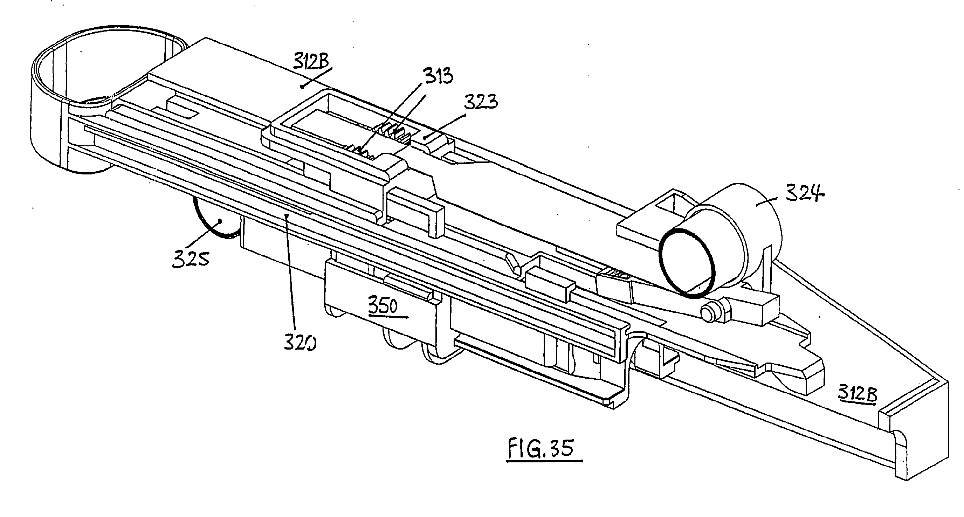

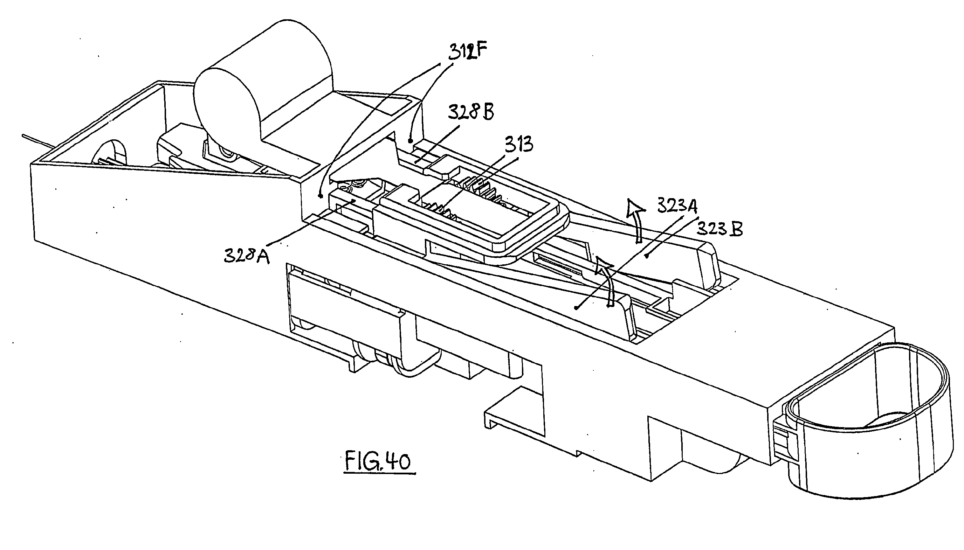

Beim dritten Ausführungsbeispiel (Figuren 21-47) wird der Gedanke des Federspeichers insofern weitergeführt, als dass sämtliche Bewegungen des Injektionsschlittens von Federspeichern veranlasst werden; das Betätigungselement umfasst hierzu eine Ladezugstange, deren Betätigung durch den Patienten vor dem Aufsetzen der Injektionsvorrichtung auf die Haut die Gesamtenergie für Einstechhub H1, Injektionshub H2 und Rückholhub H3 in diesen Federspeichern abspeichert, von denen sie dann während der Bewegungsabläufe positionsabhängig von den entsprechenden Bauteilen im Gehäuse abgerufen werden. Bei dieser Lösung ist der gesamte Ablauf auch hinsichtlich seiner Schnelligkeit und der Dauer der einzelnen Hübe H1, H2, H3 von der spezifischen Art der Betätigung durch den Patienten entkoppelt, da mit der Auslösung der Injektionsvorrichtung z. B. mittels eines Auslöseknopfes die Abläufe durch Bemessung der baulichen Parameter wie z. B. die Wahl der Federeigenschaften vorgegeben und vom Patienten nicht beeinflussbar sind. Damit kann auch hinsichtlich des Einstechhubs H1 und des Injektionshubs H2 eine Optimierung z. B. hinsichtlich deren Zeitdauer z. B. auf die Dicke der Nadel oder Injektionsvorgaben für ein spezifisches Arzneimittel umgesetzt werden.

Die konstruktive Gestaltung der wesentlichen Bauelemente ist in den Zeichnungen z. T. mehrfach dargestellt und wird daher im folgenden anhand der Funktion dieser Bauteile erläutert. Es versteht sich von selbst, dass die Detailgestaltungen der Bauteile weitgehend variabel sind, solange insbesondere gewährleistet ist, dass Anfang und Ende der Hübe H1, H2, H3 durch eine geeignete Kopplung / Entkopplung der dafür vorgesehenen Bauteile eindeutig definiert sind, und die hierfür jeweils erforderliche Energie, sei es durch unmittelbare Umsetzung der Bewegung einer Schubstange, sei es durch Abruf einer gespeicherten Energie, zeitgenau zur Verfügung gestellt wird.

### Erstes Ausführungsbeispiel

Die Baugruppen des ersten Ausführungsbeispiels sollen nun im folgenden kurz beschrieben werden:
Das Betätigungselement besteht aus einer Schubstange 120 mit rückseitiger Flanschplatte 123, die im Gehäuse 110 längs geführt ist. Auf ihrer Oberseite weist die Schubstange 120 eine Zahnung 124 auf, in die ein Zahnrad 113 greift, das in einem Schlitten 114A gelagert ist. Die Unterseite des Stössels 150 weist eine entsprechende Zahnung 154 auf, in die das Zahnrad 113 ebenfalls eingreift.

Die Drehung des Zahnrads 113 kann mittels eines Sperrschiebers 114 mit Rasthaken 119 im Schlittten 114A blockiert oder freigegeben werden: in Blockierstellung wird somit die Linearbewegung der Schubstange 120 unmittelbar in eine gleiche Linearbewegung des Stössels 150 umgesetzt, der dann entsprechend seiner Kopplung mit der Spritzenaufnahme 140 mit dieser zusammen (Einstechhub H1) oder alleine (Injektionshub H2) nach vorne fährt. Nach der Injektion wird die Sperrung des Zahnrades 113 aufgehoben und die Weiterbewegung der Schubstange 120 wird in eine gegengerichtete Verschiebung des Stössels 150 umgesetzt, der die Spritzenaufnahme 140 mitnimmt und somit die Spritze 100 mit ihrer Nadel 108 aus der Einstichstelle herauszieht.

Die Detailausgestaltung und das Zusammenwirken dieser Bauteile werden aus der folgenden Funktionsbeschreibung deutlich:
Die Spritze 100 wird mit Schutzkappe 107 in das Gehäuse 110 eingeführt und mit Ihrem Spritzenbund 102 in der Spritzenaufnahme 140 fixiert.

Nachdem die Schutzkappe 107 abgenommen und die Injektionsvorrichtung auf die Injektionsstelle aufgesetzt wurde, werden wie beim Verabreichen einer Spritze üblich, zwei Finger unter die Halteplatte 111, die formschlüssig mit dem Gehäuse 110 verbunden ist, gelegt und mit dem Daumen Kraft auf die Flanschplatte 123 der Schubstange 120 ausgeübt.

Denkbar ist auch, die Injektionsvorrichtung als komplettes Gehäuse, z.B. mit einem Klapp- oder Schiebedeckel, zu gestalten, so dass der Anwender mit einer Hand die Injektionsvorrichtung fasst, auf die Injektionsstelle aufsetzt und mit der anderen Hand auf das Betätigungselements drückt.

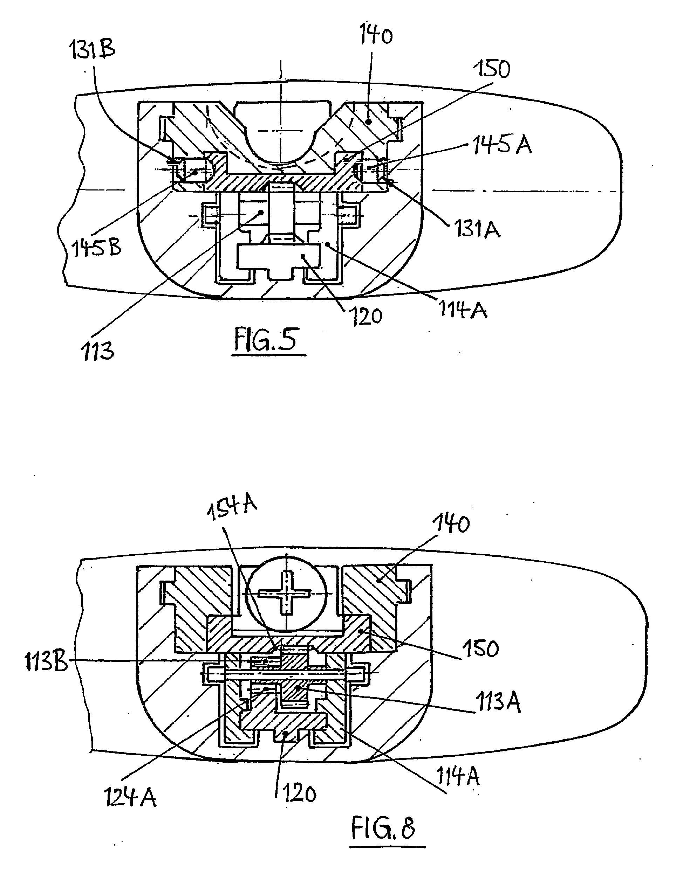

Die Spritzenaufnahme 140 und der Stössel 150 sind über Kulissensteine 145A,145B als Kopplungselement formschlüssig miteinander verbunden (siehe Figur 5).

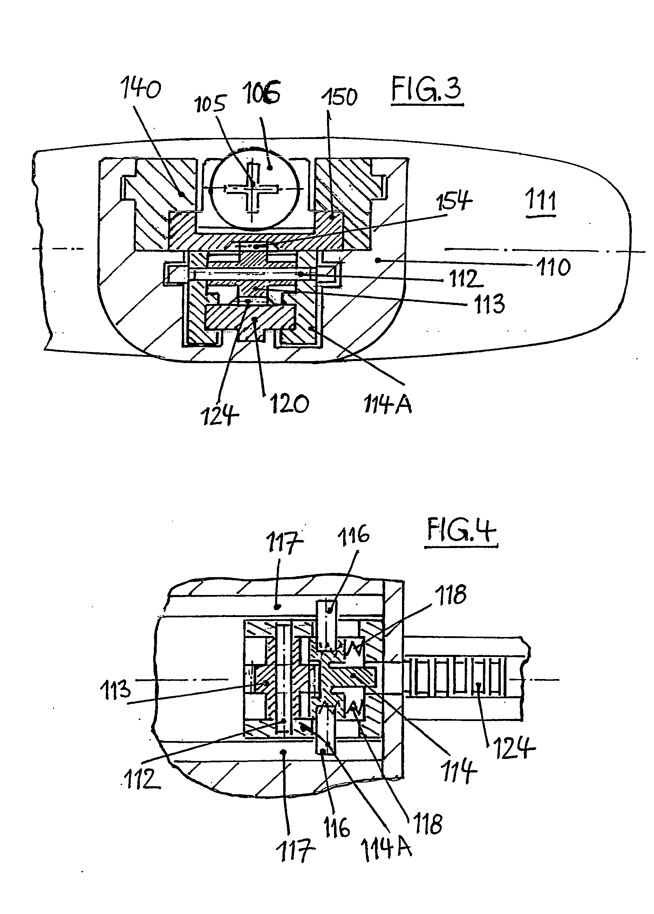

Das im Gehäuse 110 mittels einer Achse 112 im Schlitten 114A gelagerte Zahnrad 113 ist mit den Zähnen des Sperrschiebers 114 im Eingriff, so dass das Zahnrad 113 gegen Verdrehung gesperrt ist. Der Schlitten 114A ist seinerseits im Gehäuse 110 längsverschiebbar, wobei Mitnehmer 116 in einer Nut 117 gleiten. Die Zahnungen auf dem Betätigungselement 120 und dem Stössel 150 sind ebenfalls im Eingriff mit dem Zahnrad 113. Dadurch ergibt sich eine starre Verbindung zwischen Schubstange 120 und Stössel 150 (siehe Figuren 3,4).

Wird mit dem Daumen eine Kraft auf die Flanschplatte 123 ausgeübt, die größer als die Haltekraft eines Rasthakens ist, der die Spritzenaufnahme 140 im Gehäuse 110 fixiert, bewegen sich Spritzenaufnahme 140 und Stössel 150, formschlüssig verbunden über die Kulissensteine 145A,145B synchron zur Schubstange 120 zur Injektionsstelle hin. Die Nadel 108 sticht in das Gewebe bis zur vorbestimmten Tiefe (Einstechhub H1) ein, ohne dass der Kolben 104 der Spritze 100 betätigt wird.

Am Ende des Einstechhubes H1 erreichen die Kulissensteine 145A, 145B Ausnehmungen 115A, 115B im Gehäuse 110. Bedingt durch die Kraftumsetzung von Abschrägungen 131A, 131 B gleiten die Kulissensteine 145A, 145B in ihre zugeordnete Ausnehmung 115A,115B, fixieren die Spritzenaufnahme 140 formschlüssig im Gehäuse 110 und heben dadurch die starre Kopplung zwischen Spritzenaufnahme 140 und Stössel 150 auf.

Weiter angetrieben durch die Schubstange 120, wird nun der Stössel 150 weiter zur Injektionsstelle hin bewegt, über den Flansch 106 und Kolbenstange 105 wird der Kolben 104 im Spritzenkörper 101 bewegt und dadurch das Arzneimittel injiziert (Injektionshub H2).

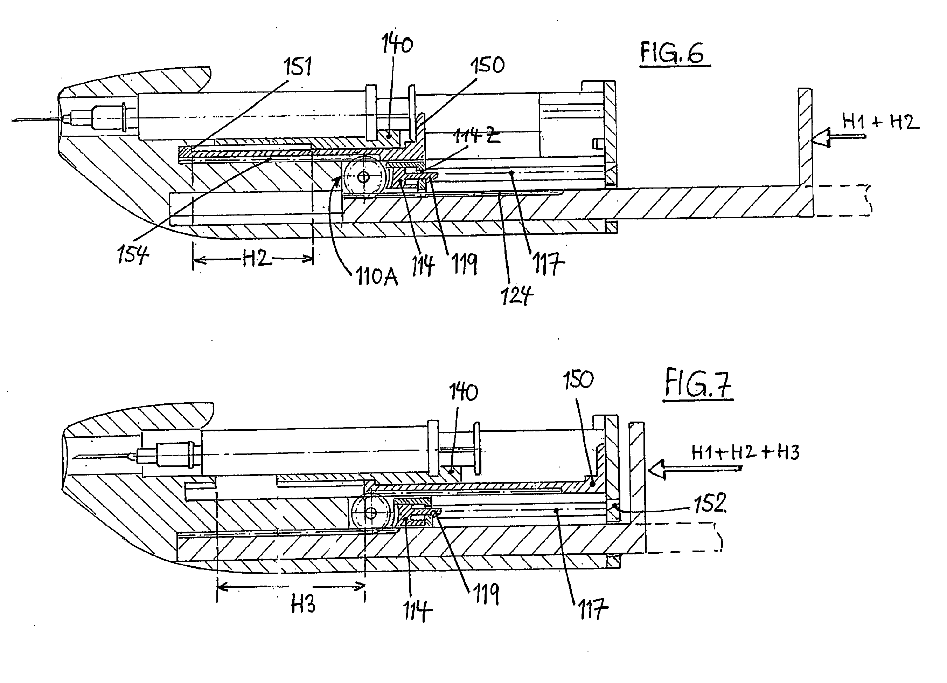

Am Ende des Injektionshubes H2 schlagen die Mitnehmer 116 beidseitig des Sperrschiebers 114 am Ende der Nut 117 an. Der Sperrschieber 114 wird gegen die Federkraft von zwei Druckfedern 118 verschoben, die Sperrung des Zahnrades 113 wird freigegeben, der Rasthaken 119 rastet in eine Öffnung 114 Z des Schlittens 114 A ein. Anschließend trifft der Schlitten 114A des Zahnrads 113 auf einen Anschlag 110A im Gehäuse 110 (siehe Figur 6).

Da in dieser Position das Zahnrad 113 entsperrt ist, und der Schlitten 114 A sich auf den Betätigungselement 120 axial verschieben lässt, erfolgt beim weiteren Vorschieben des Betätigungselements 120 in Richtung Injektionsstelle eine Drehung des Zahnrades 113. Der Stössel 150 bewegt sich dadurch von der Injektionsstelle weg, ohne dass die Kolbenstange 105 bewegt wird.

Hat der Stössel 150 einen Weg, der betragsmäßig dem Injektionshub H2 entspricht, zurückgelegt, wird über einen Anschlag 151 die Spritzenaufnahme 140 mitgenommen, die Kulissensteine 145A, 145B verschieben sich und koppeln den Stössel 150 wieder mit der Spritzenaufnahme 140, so dass nun über den Spritzenbund 102 die Spritze 100 und damit die Nadel 108 um einen Rückholhub H3, der betragsmäßig dem Einstechhub H1 entspricht, zurückgezogen wird. (siehe Figur 7)

Der Abstand zwischen der Flanschplatte 123 des Betätigungselements 120 und der Halteplatte 111 lässt sich nun nicht mehr verringern, Spritzenaufnahme 140 und Stössel 150 sind in Ihre Ausgangslage zurückgesetzt.

Die Spritze 100 kann nun unmittelbar entnommen werden, oder die Schubstange 120 kann zunächst in seine Ausgangslage zurückgezogen werden und dann die Spritze entnommen werden.

Beim Zurückziehen der Schubstange 120 wälzt sich das Zahnrad 113 auf der Zahnung 154 des Stössels 150 und der Zahnung 124 der Schubstange 120 ab.

Der Schlitten 114 A bewegt sich dabei relativ zur Schubstange 120.

Kurz vor Ende des Rückzuges der Schubstange 120 bewegt sich der Rasthaken 119 gegen eine Schräge 152, dadurch wird die Verrastung freigegeben, Druckfedern 118 schieben den Sperrschieber 114 wieder gegen das Zahnrad 113. Damit wird das Zahnrad 113 wieder gegen Drehung gesperrt und wieder eine starre Verbindung zwischen Schubstange 120 und Stössel 150 erreicht.

Bei einer ersten Variante dieses Ausführungsbeispiels (Fig. 8) sind im Schlitten 114A zwei Zahnräder 113A, 113B als Getriebe ausgebildet, so dass ein Übersetzungsverhältnis der Bewegungen von Schubstange 120 und Stössel 150 definiert ist, das den Weg der Schubstange 120 verkürzt, und/oder einen schnelleren Rückhub ermöglicht.

Das größere Zahnrad 113A kämmt mit der Zahnung 154A des Stössels 150, das kleinere Zahnrad 113B mit der Zahnung 124A der Schubstange 120.

Sobald das Zahnradpaar 113A, 113B entsperrt wird (am Ende des Injektionshubes H2), wird der Rückholhub H3 des Stössels 150 im Verhältniss der Teilkreisdurchmesser der beiden Zahnräder 113A, 113B übersetzt.

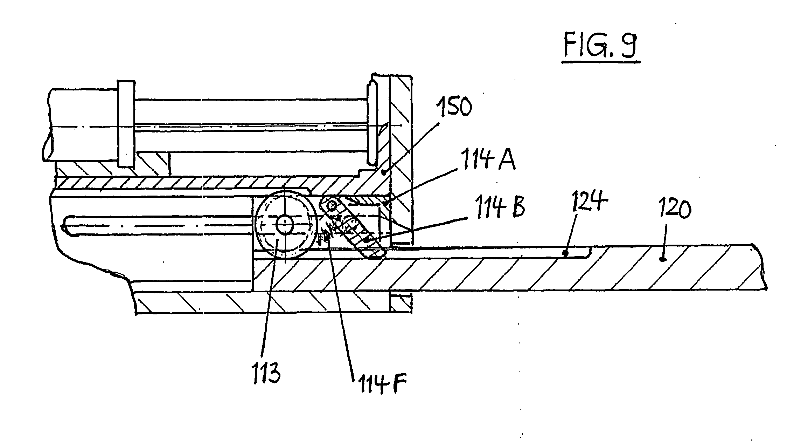

Bei einer zweiten Variante des ersten Ausführungsbeispiels (Fig. 9) ist zur Sperrung / Freigabe des Zahnrads 113 ein mit einer Feder 114F beaufschlagter Hebel 114B vorgesehen.

Die Funktion des Sperrschiebers 114 wird hierbei durch eine lösbare Fixierung des Schlittens 114A, in dem das Zahnrad 113 gelagert ist, auf der jeweiligen Zahnung der Schubstange 120 und/oder des Stössels 150 erreicht.

Der Hebel 114B, der im Schlitten 114A drehbar gelagert ist, greift an seiner der Lagerstelle gegenüberliegenden Seite in die Verzahnung 124 der Schubstange 120 ein.

Solange der Hebel 114B ein Verschieben des Schlittens 114A auf der Schubstange 120 verhindert, ergibt sich eine starre Verbindung zwischen der Schubstange 120 und dem Stössel 150.

Gegen Ende des Injektionshubes H2 trifft ein Mitnehmer auf den Endanschlag der Nut 117, der Hebel 114B wird gegen die Zugkraft der Feder 114F aus der Verzahnung 124 der Schubstange 120 ausgeschwenkt, gleichzeitig trifft der Schlitten 114A auf den Anschlag 110A (siehe Figur 6), das Zahnrad 113 kann sich nun drehen und der Rückholhub H3 beginnt.

Anstelle dieser Lösung ist auch die Lagerung eines federbeaufschlagten Schwenkhebels im Schlitten 114 A möglich, dessen Sperrklinke in die Verzahnung des Zahnrads 113 eingreift.

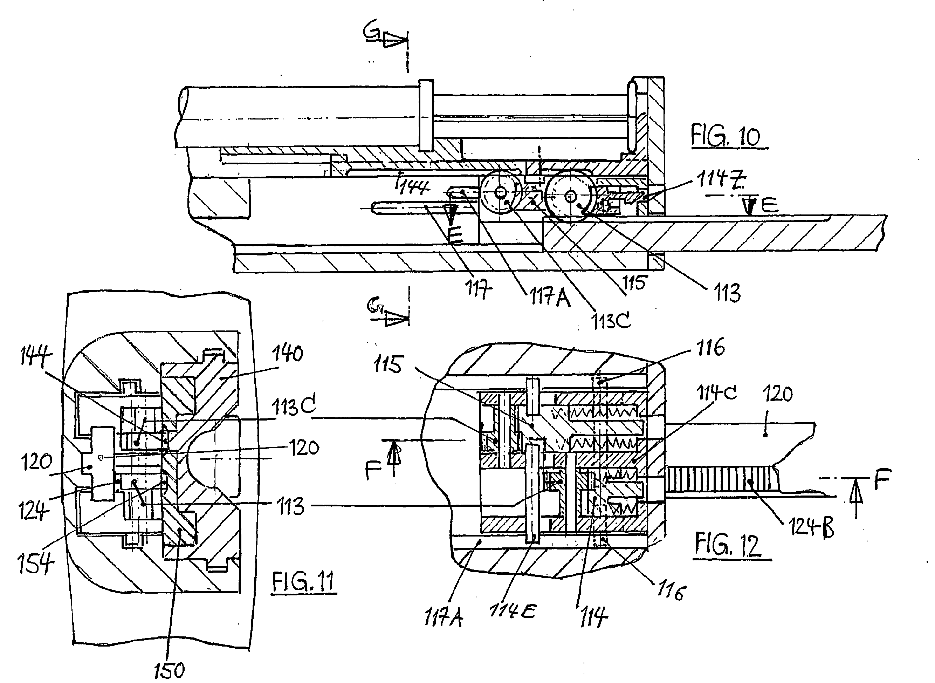

Eine dritte Variante dieses Lösungsprinzips des ersten Ausführungsbeispiels ist in den Figuren 10-12 dargestellt:
Bei dieser Variante des ersten Ausführungsbeispiels ist zur Koppelung zwischen Stössel 150 und Spritzenaufnahme 140, die zusammen den Injektionsschlitten bilden, ein weiteres Zahnrad 113C vorgesehen, das in einem gemeinsamen Schlitten 114C gelagert ist, der ebenfalls von der Schubstange 120 verschoben wird.

In der Ausgangsstellung ist das Zahnrad 113C durch einen weiteren Sperrschieber 115, das Zahnrad 113 durch den Sperrschieber 114 gesperrt.

Das Zahnrad 113C kämmt mit einer Zahnung 144 auf der Spritzenaufnahme 140, das Zahnrad 113 kämmt, wie oben beschrieben, mit der Zahnung 124 auf dem Betätigungselement 120 und der Zahnung 154 im Stössel 150.

Bei Bewegung der Schubstange 120 wird infolge der gesperrten Zahnräder 113, 113C eine starre Verbindung der Schubstange 120 mit der Spritzenaufnahme 140 und dem Stössel 150 erreicht.

Spritzenaufnahme 140, Schlitten 114C, und Stössel 150 werden deshalb simultan mit der Schubstange 120 zur Injektionsstelle hin bewegt, bis Mitnehmer 114E in der Nut 117A anschlagen und das Zahnrad 113C durch Verschieben des Sperrschiebers 115 entsperren. Das Zahnrad 113C kann sich nun drehen, die Spritzenaufnahme 140 wird nicht weiter bewegt.

Das Zahnrad 113 bleibt noch gesperrt, somit wird der Stössel 150 gleichförmig mit der Schubstange 120 bewegt, bis Mitnehmer 116 den Anschlag in der Nut 117 erreichen. Dann erfolgt die Bewegungsumkehr wie oben beschrieben.

Sobald die Schubstange 120 wieder in ihre Ausgangslage zurückgezogen wird, werden beide Zahnräder 113, 113C wieder gesperrt.

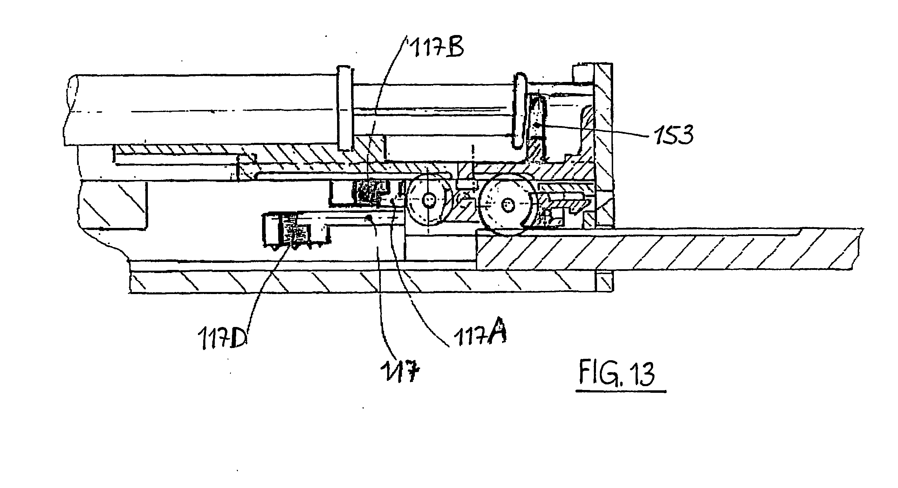

Eine vierte Variante dieses Lösungsprinzips des ersten Ausführungsbeispiels ist in Figur 13 dargestellt:
Der Weg der Mitnehmer 114E bis zum Anschlag in der Nut 117A bestimmt den Einstechhub H1.

Der Anschlag in der Nut 117A ist durch einen Schieber 117B veränderbar. Somit kann die Einstechtiefe in einem bestimmten Bereich variabel eingestellt werden. So könnte z. B. mit einer 16 mm Kanüle (16 mm lange Nadel) durch Verschieben des Schiebers 117B eine Einstechtiefe von nur 12 mm erreicht werden.

In gleicher Weise kann der Injektionshub H2 durch einen veränderbaren Anschlag 117D in der Nut 117 erfolgen.

Durch eine Gestaltung des Stössels 150 mit einem oder mehreren u-förmigen Stegen 153 ist es durch ein derartiges Prinzip einer längeneinstellbaren Nut möglich, unterschiedliche Injektionsvolumina zu verabreichen.

### Zweites Ausführungsbeispiel

Das zweite Ausführungsbeispiel ist in den Figuren 14-20 dargestellt; die Baugruppen des zweiten Ausführungsbeispiels sollen nun im folgenden kurz beschreiben werden:
Zusätzlich zu den oben beschriebenen Bauteilen (Schubstange 220, Spritzenaufnahme 240 und Stössel 250), ist ein Rückholschlitten 260 mit dem Injektionsschlitten gekoppelt, der mittels Druckfedern 261A, 261 B sich an einer Anschlagwandung des Gehäuses 210 abstützt.

Die Hube H1 und H2 werden von einem in der Schubstange 220 verschwenkbar gehaltenen, federbeaufschlagen Steuerhebel 221 in ihrem Ablauf gesteuert. Am Ende des Injektionshubs H2 gibt die Schubstange 220, wie unten beschrieben, den nunmehr gegen die Druckfedern 261 A, 261 B vorgespannten Rückholschlitten 260 frei, der dann den Rückholhub H3 selbstätig durchführt.

Die Spritze 200 wird mit Schutzkappe 207 durch eine Schwenkbewegung in das Gehäuse 210 eingeführt und mit Ihrem Spritzenbund 202 in der Spritzenaufnahme 240 und mit dem Flansch 206 der Kolbenstange 205 im Stössel 250 fixiert.

Nachdem die Schutzkappe 207 abgenommen und die Injektionsvorrichtung auf die Injektionsstelle aufgesetzt wurde, werden auch hier zwei Finger unter die Halteplatte 211 gelegt und mit dem Daumen Kraft auf die Flanschplatte 223 des Betätigungselements 220 ausgeübt.

Die Schubstange 220 ist an ihrem vorderen Ende mit Abschrägungen 225 versehen, die gegen Rastzungen 262A,262B des Rückholschlittens 260 drücken. Die durch die Abschrägungen 225 auf die Rastzungen 262 wirkende radiale Kraftkomponente stützt sich an der Gehäusewand ab. Dadurch verschiebt sich der Rückholschlitten 260 gegen die Kraft der Druckfedern 261 A, 261 B zur Injektionsstelle hin.

Die Spritze verbleibt jedoch in Ihrer Lage, da die Spritzenaufnahme 240 und der Stössel 250 zu diesem Zeitpunkt nicht mit der Schubstange 220 gekoppelt sind.

Die Spritzenaufnahme 240 ist gegen ein ungewolltes Verschieben durch Reibungskräfte oder die Gewichtskraft bei einer senkrechten Injektion durch Rastzungen 241 an der Spritzenaufnahme 240, die in das Gehäuse 210 eingreifen, gesichert. Ebenso ist der Stössel 250 durch die Rastzungen 251 gesichert, die ebenfalls in das Gehäuse 210 eingreifen.

Hat nun die Schubstange 220 den Weg zur Spannung des Rückholschlittens 260 durchgeführt, können die Rastzungen 262A, 262B in die Ausnehmungen 212A, 212B im Gehäuse 210 ausweichen, der Formschluss zwischen den Rastzungen 262A, 262B und der Schubstange 220 wird aufgehoben und der Rückholschlitten 260 formschlüssig in Gehäuse 210 fixiert.

Während der Spannung des Rückholschlittens 260 bewegt sich der Steuerhebel 221, der durch eine Flachfeder 222 mit einem rechtsdrehenden Moment beaufschlagt wird (welches jedoch nicht zu einer Drehung führt, da sich der Steuerhebel 221 in einer Nut 213 im Gehäuse 210 abstützt), bis an fluchtende Wandungen eines ersten Anschlags 242 der Spritzenaufnahme 240 und eines zweiten Anschlags 252 des Stössels 250.

Die über den Daumen fühlbare Kraft an der Schubstange 220 nimmt während des Spannhubes des Rückholschlittens infolge der Federkennlinie der Druckfedern 261 A, 261 B linear zu.

Die im Moment des Ausweichens der Rastzungen 262A, 262B in die Ausnehmungen 212A, 212B durch den Daumen wirkende Kraft wird nun über den Steuerhebel 221 über den ersten Anschlag 242 auf die Spritzenaufnahme 240 und den Stössel 250 übertragen.

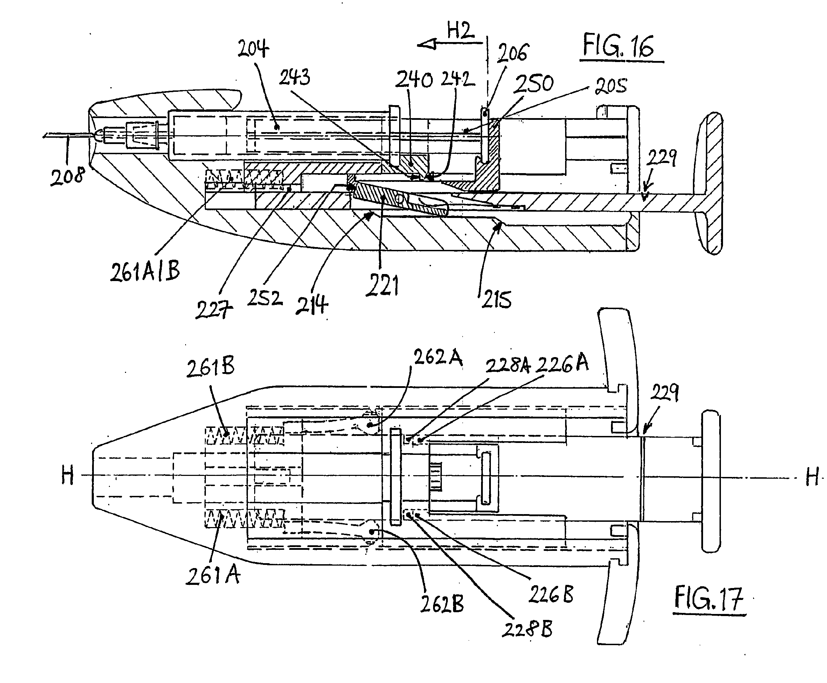

Die Rastzungen 241, 251 weichen aus, Spritzenaufnahme 240 und Stössel 250 bewegen sich mit der Spritze gleichförmig, jedoch durch den Kraftimpuls schlagartig in Richtung Injektionsstelle. Die Nadel 208 bewegt sich dabei um den Einstechhub H1 (Figur 16).

Am Ende des Einstechhubes H1, der kleiner oder gleich dem Spannweg sein muss, wird der Steuerhebel 221 durch eine erste Abschrägung 215 in der Nut 213 gegen den Uhrzeigersinn gedreht und dadurch der Formschluss von Steuerhebel 221 und der Spritzenaufnahme 240 am ersten Anschlag 242 gelöst.

Da der Formschluss mit dem Stössel 250 über den zweiten Anschlag 252 erhalten bleibt, wird nun beim Weiterschieben der Schubstange 220 über den Flansch 206 und die Kolbenstange 205 der Kolben 204 der Spritze 200 bewegt und das Arzneimittel injiziert.

Sobald das Ende des Injektionshubs H2 erreicht ist, wird der Steuerhebel 221 durch eine zweite Abschrägung 214 um weitere Winkelgrade gegen den Uhrzeigersinn gedreht und damit auch der Formschluss zwischen dem zweiten Anschlag 252 des Stössels 250 und dem Steuerhebel 221 gelöst.

Gleichzeitig oder auch nach Zurücklegung eines zusätzlichen Weges der Schubstange 220 weichen die Rastzungen 262A, 262B in Ausnehmungen 226A,226B an der Schubstange 220 aus. Dadurch wird der Formschluss zwischen Rückholschlitten 260 und Gehäuse 210 aufgehoben und infolge der Kraft der Druckfedern 261A, 261 B der Rückholschlitten 260, die Spritzenaufnahme 240, der Stössel 250 und damit die Spritze 200 von der Injektionsstelle weg bewegt.

Die Nadel 208 wird zwangsläufig aus dem Körper herausgezogen und die Spritze 200 in ihre Ausgangslage gebracht.

Bei diesem Vorgang ändert sich die Lage der Schubstange 220 nicht.

Die Spritze 200 kann danach entnommen werden, oder aber die Schubstange 220 kann in ihre Ausgangslage zurückgezogen und dann die Spritze entnommen werden.

Beim Zurückziehen der Schubstange 220 in ihre Ausgangsposition nimmt ein Anschlag 227 den Stössel 250 und dieser über einen Anschlag 243 die Spritzenaufnahme 240 in ihre Ausgangsposition mit. Gleichzeitig werden die Rastzungen 262A, 262B mittels Schrägen 228A, 228B nach oben ausgelenkt, gleiten über die Schubstange 220 hinweg und rasten hinter der Schubstange 220 ein, sobald diese ihre Endlage erreicht hat.

Über eine Markierung 229 kann optisch kontrolliert werden, ob sich die Schubstange 220 wieder in ihrer Ausgangsposition befindet.

### Drittes Ausführungsbeispiel

Ein Gesamtschnitt des dritten Ausführungsbeispiels ist in Figur 21 dargestellt.

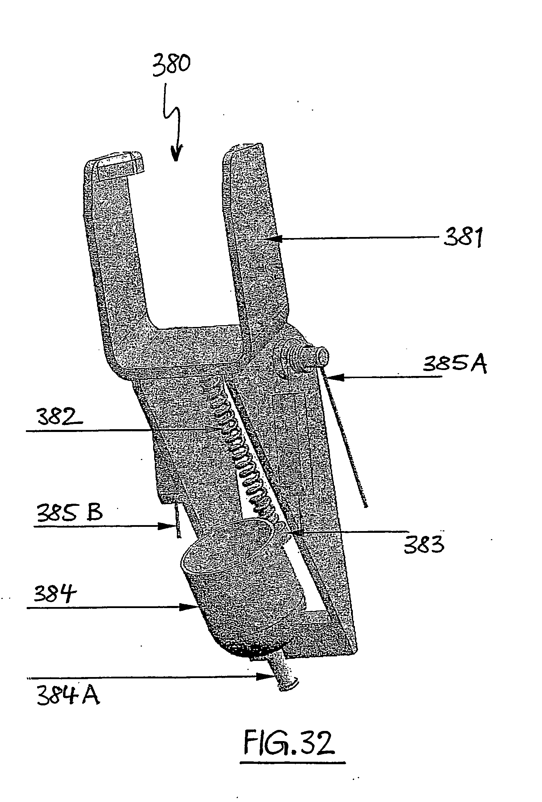

Die Baugruppen des dritten Ausführungsbeispiels sollen nun zunächst kurz beschrieben werden:
Das Gehäuse 310 weist an seinem vorderen Ende (Injektionsende) einen nach unten zeigenden Griff auf, der zur einfachen Handhabung dient, und in dem auch ein Klingelmechanismus (Fig. 32) untergebracht werden kann, der das Ende der vollautomatisch ablaufenden Hube H1, H2, H3 akustisch anzeigt.

Anstelle der Schubstangen 120, 220 bei den beiden vorhergehend erläuterten Ausführungsbeispielen tritt hier als wesentliches Betätigungselement eine Ladezugstange 320, durch die eine Vorschubfeder 324 gespannt wird, die der Vorschubbewegung und der Rückholbewegung des Injektionsschlittens dient.

Die Vorschubfeder 324 wird durch Steuerelemente z. B. einen Auslösehebel 326 freigegeben.

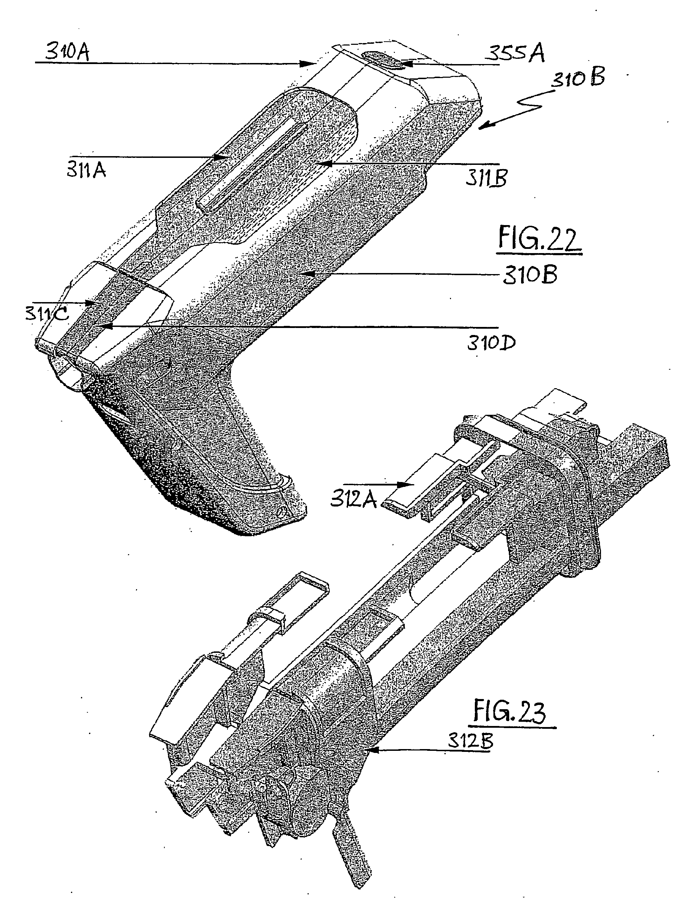

Der Aufbau des Gehäuses 310 ist in den Figuren 22 und 23 dargestellt: Das Gehäuse 310 selbst ist zweiteilig ausgeführt mit zwei Gehäuseschalen 310A,310B und einer zweiteiligen Abdeckung 311 A,311 B über der Spritze 300, die nach Beendigung der Injektion geöffnet werden kann, sowie mit einer Öffnung für eine Signalfläche 355A zur Anzeige des Ladezustands.

Innerhalb des Gehäuses 310 ist ein ebenfalls zweiteiliger Aufnahmerahmen 312 mit zwei symmetrischen Hälften 312A,312B gehalten, in dem die beweglichen Funktionsteile axial verschiebbar sind, und in dem auch die Betätigungselemente aufgenommen sind.

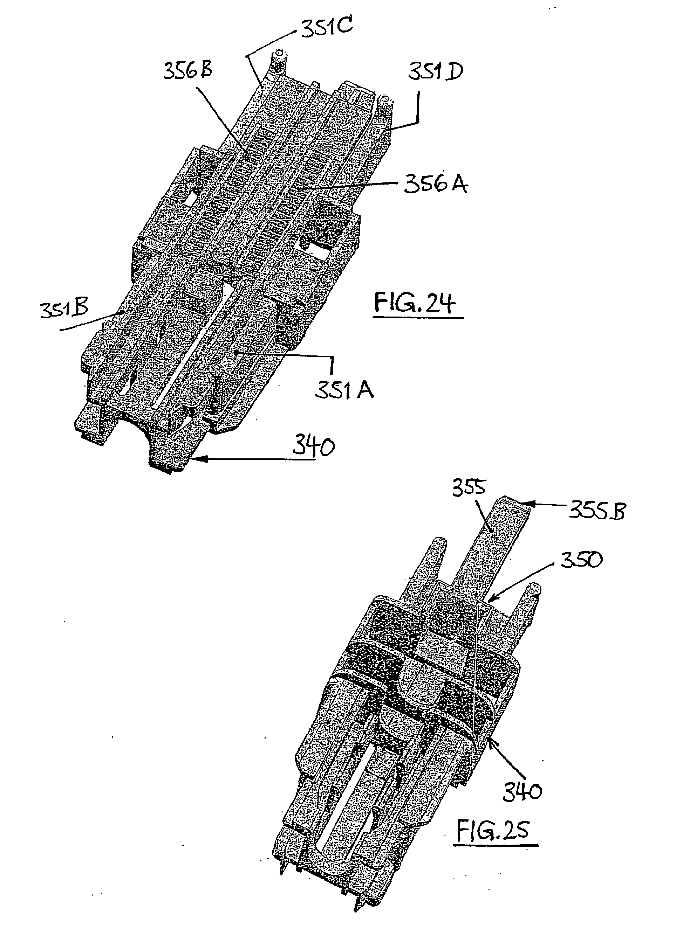

Figur 24 und 25 zeigen den Injektionsschlitten, bestehend aus Spritzenaufnahme 340 und Stössel 350; letzterer weist einen rückwärtigen Fortsatz 355 auf, dessen Stirnseite 355B die oben genannte Signalfläche 355A des Gehäuses 310 bildet.

Der Stössel 350 weist seitliche Verriegelungsarme 351A, 351 B für den Spritzenvorschub auf. Wie bei allen Ausführungsbeispielen sind Spritzenaufnahme 340 und Stössel 350 ineinander verschiebbar, damit der Stössel 350 den Injektionshub H2 durchführen kann. Auf der Unterseite des Stössels 350 sind zwei Zahnungen 356A, 356B zum Vorschub des Stössels 350 relativ zur Spritzenaufnahme 340 zu sehen.

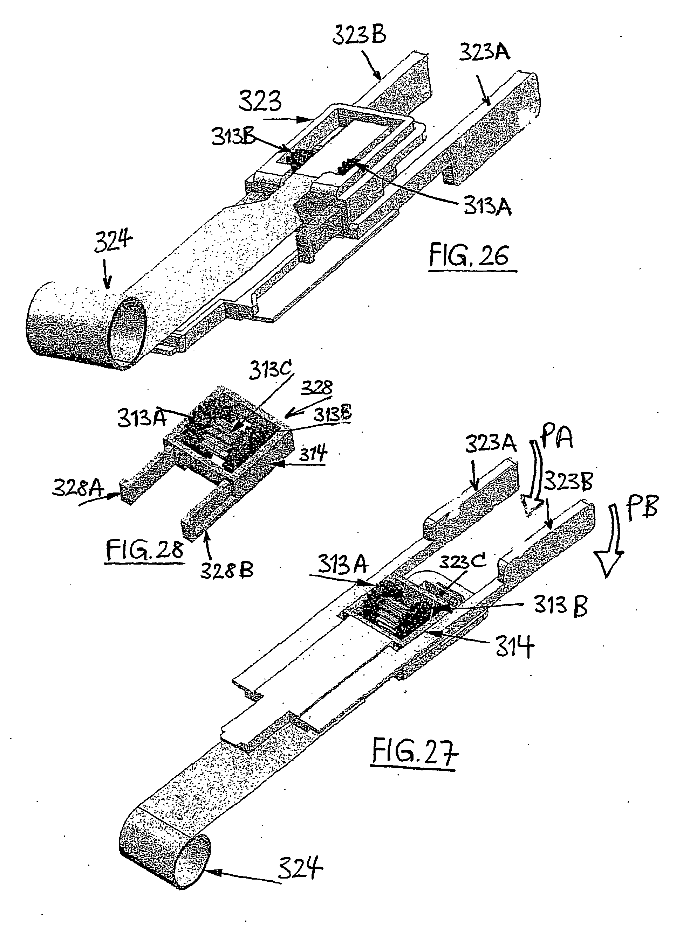

Die Figuren 26-28 zeigen in Aufsicht (Figur 26) und Unteransicht (Figur 27) ein weiteres Bauteil des Betätigungselements, den Vorschubschlitten 323, mit einem Zahnradgetriebe 328 in einem Gehäuse 314, dessen Doppelzahnrad 313A,313B einerseits in die Zahnungen 356A, 356B des Stössels 350 eingreifen, und dessen mittleres Zahnrad 313C mit einer Zahnung 323 eines Vorschubschlittens 323 zusammenwirkt. An einem Ende weist der Vorschubschlitten 323 zwei seitlich herausragende Verriegelungsarme 323A, 323B auf, die soweit elastisch sind, dass sie in Richtung der Pfeile PA,PB nach unten verschwenkbar sind. Am anderen Ende ist eine Rollfeder als Vorschubfeder 324 angeordnet, die in Längsrichtung am Vorschubschlitten 323 angreift. Das Zahnradgetriebe 328 weist ferner in Richtung der Vorschubfeder 324 zeigende seitliche Anschlagstangen 328A, 328B auf.

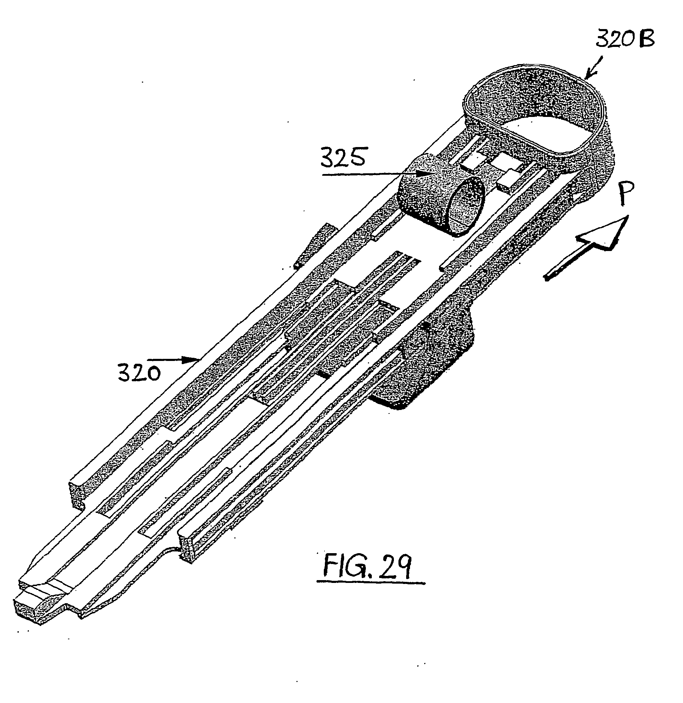

Figur 29 zeigt ein weiteres wesentliches Bauteil des Betätigungselements, die Ladezugstange 320, mit einer Rückzugsfeder 325 und einem Griff 320B, der aus dem Gehäuse 310 nach außen ragt. Durch Ziehen der Ladezugstange 320 in Richtung des Pfeiles P gegen die Kraft der Vorschubfeder 324 wird der Injektionsschlitten (Spritzenaufnahme 340 und Stössel 350) in seine Ausgangsposition gebracht und gespannt. Die jetzt ebenfalls gespannte Rückzugsfeder 325 bringt nach dem Loslassen des Griffs 320B die Ladezugstange 320 automatisch in ihre Ausgangsposition zurück. Durch Betätigung eines Auslösemechanismus 370 wird die gespeicherte Energie der Vorschubfeder 324 an Spritzenaufnahme 340 bzw. Stössel 350 abgeben.

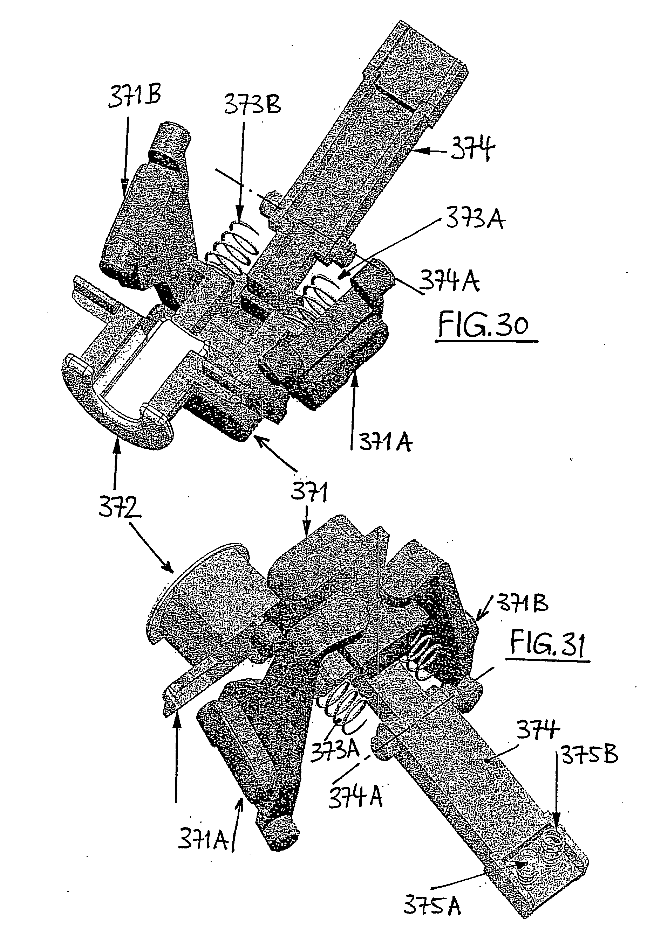

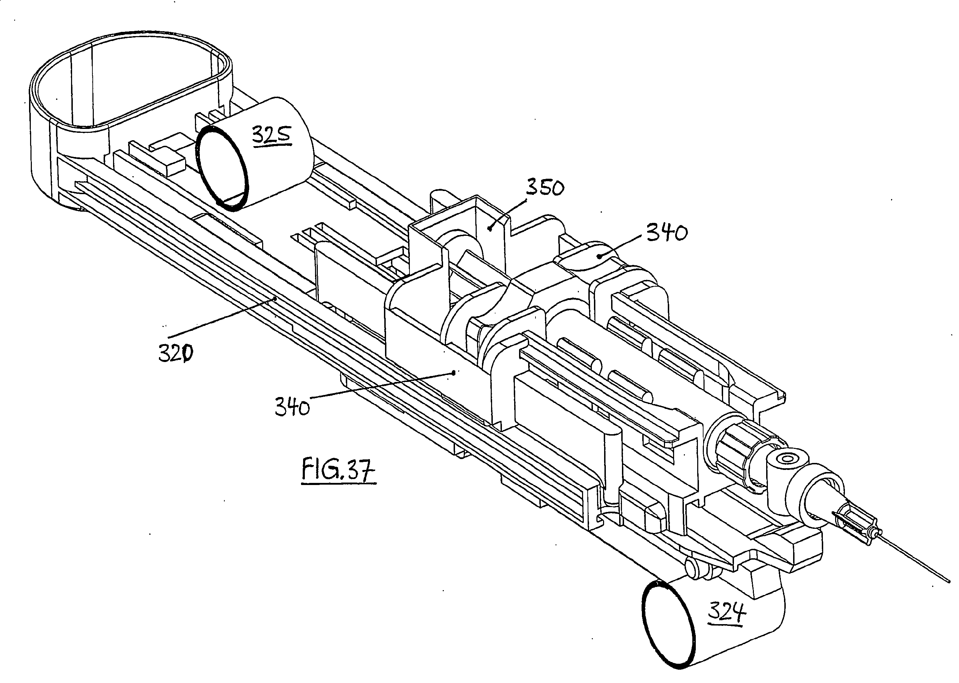

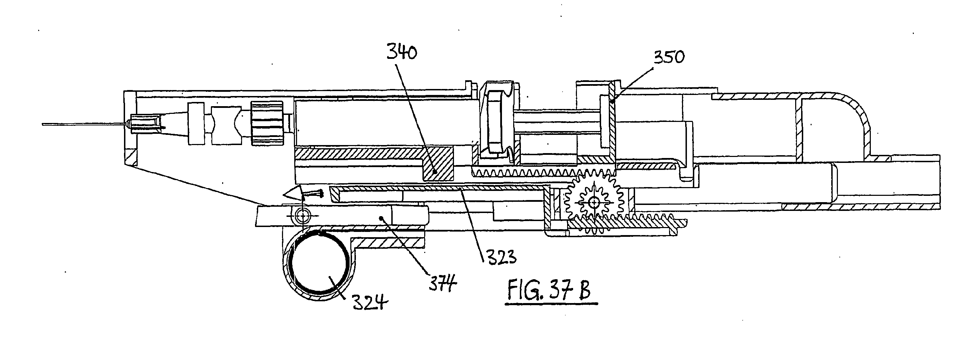

Die Figuren 30 und 31 zeigen diesen Auslösemechanismus 370 der Injektionsvorrichtung, der eine mechanische Wechselwirkung mit den Bauteilen zur Freischaltung der Vorschubfeder 324 im geladenen Zustand herstellt. Der Auslösemechanismus besteht aus einem dreiteiligen Schalter, mit einem mittleren Schaltelement 371 und zwei seitlichen Schaltflügeln 371 A, 371 B, einer ringförmigen Sicherheitskappe 372, die die Nadel 308 umschließt, und die gegen die Kraft zweier Druckfedern 373A, 373B axial im Gehäuse 310 verschiebbar ist. Erst in ihrer durchgedrückten (nicht dargestellten) Position beim Aufsetzen der Injektionsvorrichtung auf die Haut ermöglicht die Sicherheitskappe 372 durch Freigabe des Schaltelements 371 und der Schaltflügel 371A, 371 B die Betätigung eines um eine Achse 374A schwenkbaren Auslöseschwenkhebels 374 gegen zwei Druckfedern 375A, 375B. Bei Betätigung des Schaltelements 371 schwenkt dieses gegen das eine Ende des Auslöseschwenkhebels 374, dessen anderes Ende dann von der Stirnkante des vorgespannten Vorschubschlittens 323 weggeschwenkt wird, worauf der Einstechhub H1 beginnen kann (Figur 37B).

Figur 32 zeigt den Klingelmechanismus 380, dessen Klingelhebel 381 nach Beendigung des Rückholhubs H3 gegen Federn 385A,385B vorgespannt wird und nach Auslösung über einen an einer Druckfeder 382 aufgehängten Klöppel 383 an eine mit einem Haltestift 384A befestigte Glocke 384 anschlägt.

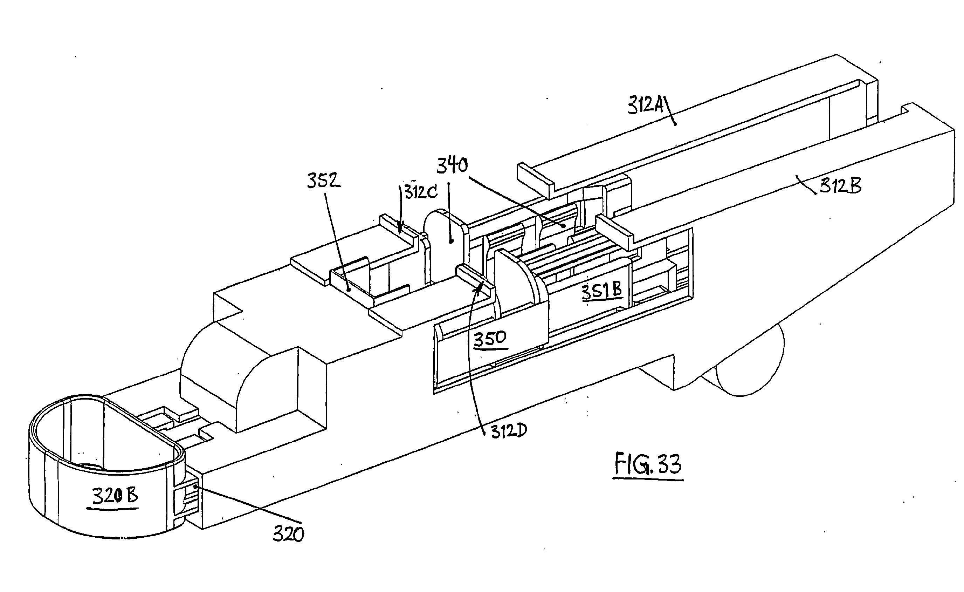

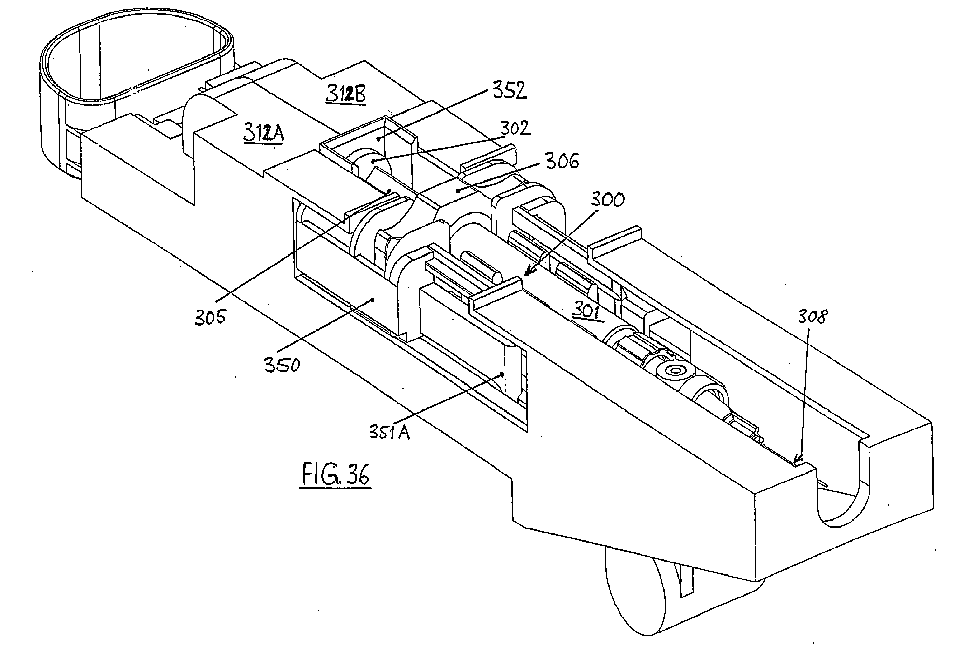

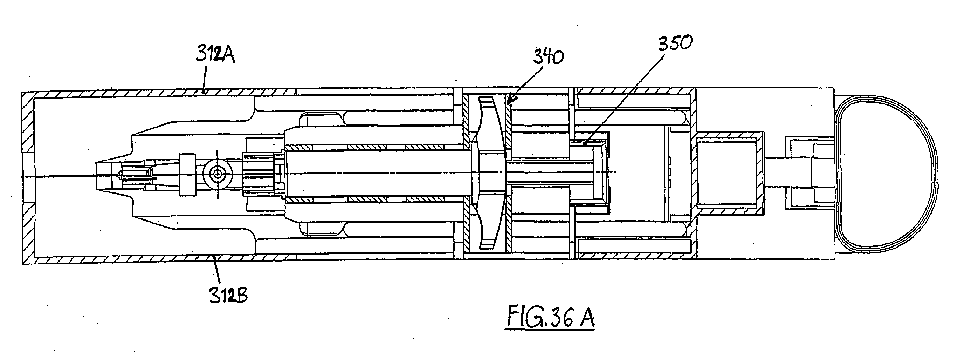

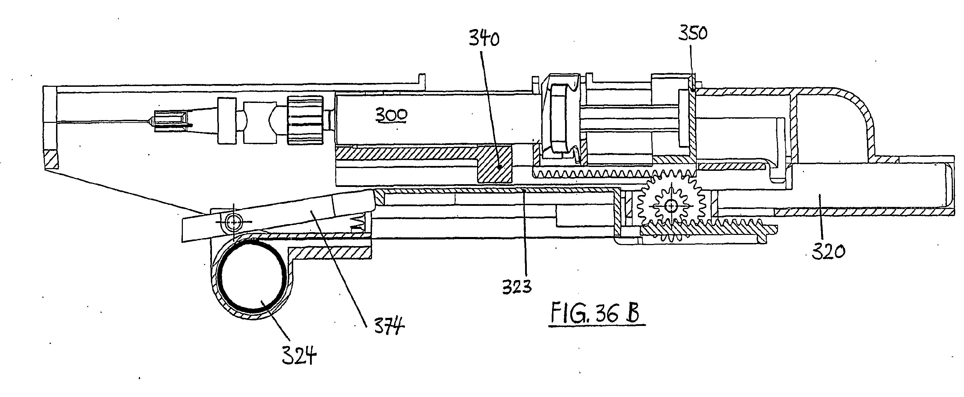

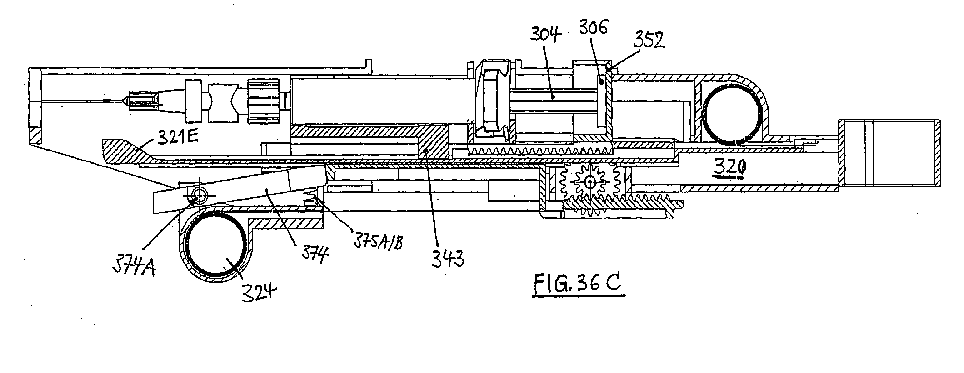

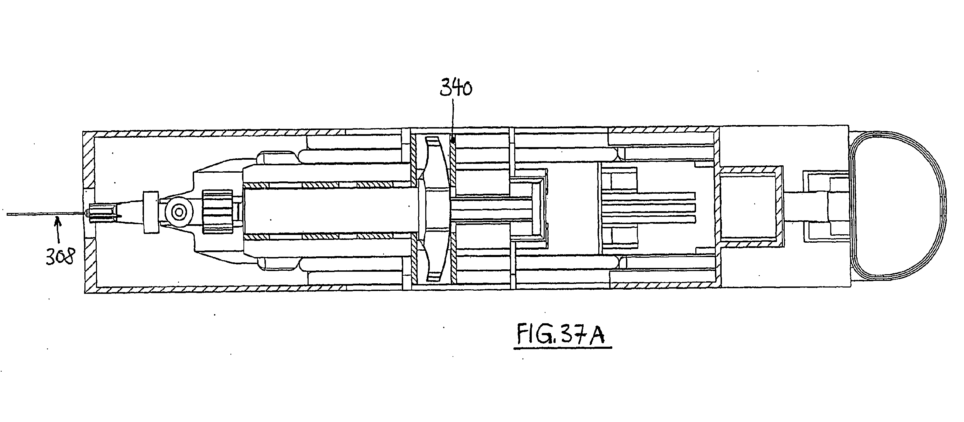

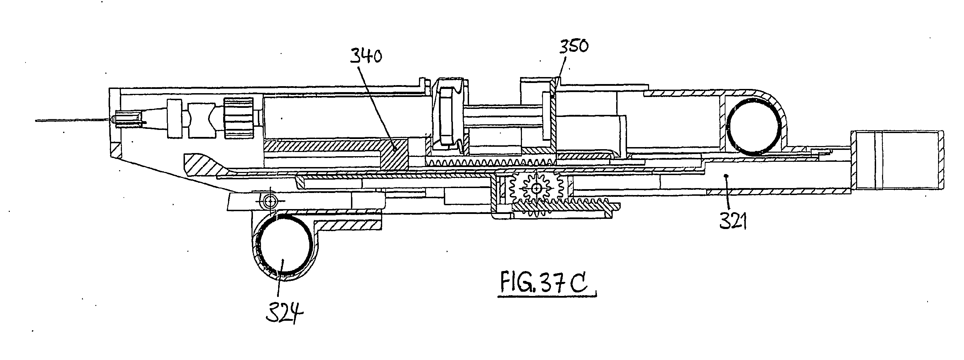

Die Funktionsweise der die beschriebenen Bauteile enthaltenen Injektionsvorrichtung ist wie folgt:
Der Injektionsschlitten mit der Spritzenaufnahme 340 und dem rahmenartig ausgebildetem Stössel 350 (Figur 24,25) befindet sich in der in den Figuren 33-37 in verschiedenen Darstellungen gezeigten Startposition an einem hinterem Anschlag 312C und 312D des Aufnahmerahmens 312A,312B, eine mit Arzneimittel gefüllte Spritze 300 wird eingelegt (Figur 36,37).

Der Einstechhub H1 der Spritze 300 (Figur 37) wird durch Betätigung des Auslösemechanismus 370 (Figur 30,31) ausgelöst, wodurch die Vorschubfeder 324 den Vorschubschlitten 323 in Injektionsrichtung zieht. Stössel 350 und Vorschubschlitten 323 stehen zunächst über das Zahnradgetriebe 328 und die Verriegelungsarme 351A, 351 B bzw. 323A, 323B miteinander in starrer Verbindung. Die Verriegelungsarme sind geführt und können erst bei Erreichen einer vorgegebenen Axialposition des Vorschubschlittens 323 durch Freischaltung durch den Aufnahmerahmen 312A,312B oder die Ladezugstange 320 und/oder die Spritzenaufnahme 340 seitlich ausgelenkt werden: die Verriegelungsarme 351A, 351 B werden durch das Ende einer Führungswand an der Ladezugstange 320 freigeschaltet, wenn die Spritzenaufnahme 340 einen vorderen Anschlag am Aufnahmerahmen 312A,312B erreicht hat.

Die Spritzenaufnahme 340 mit der Spritze 300 fährt daraufhin vor und die Nadel 308 dringt in die Haut ein (Einstechhub H1).

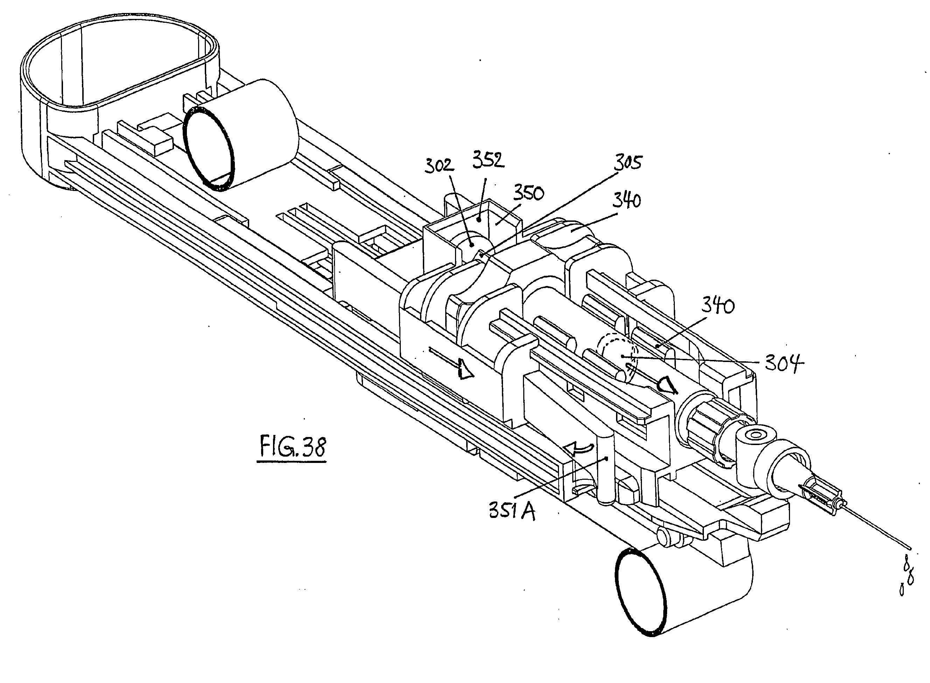

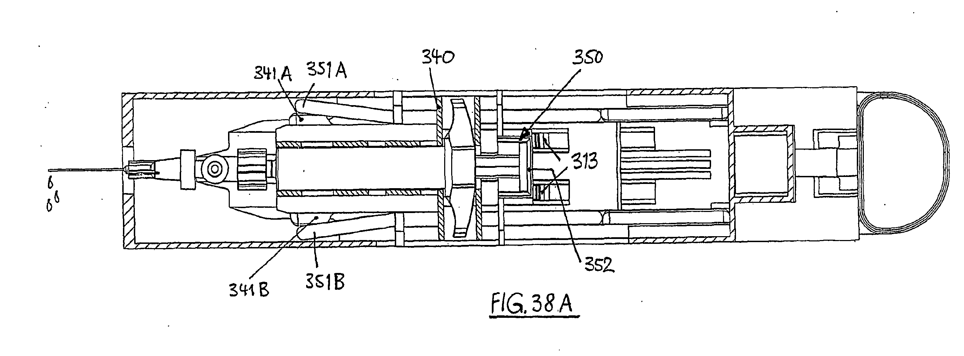

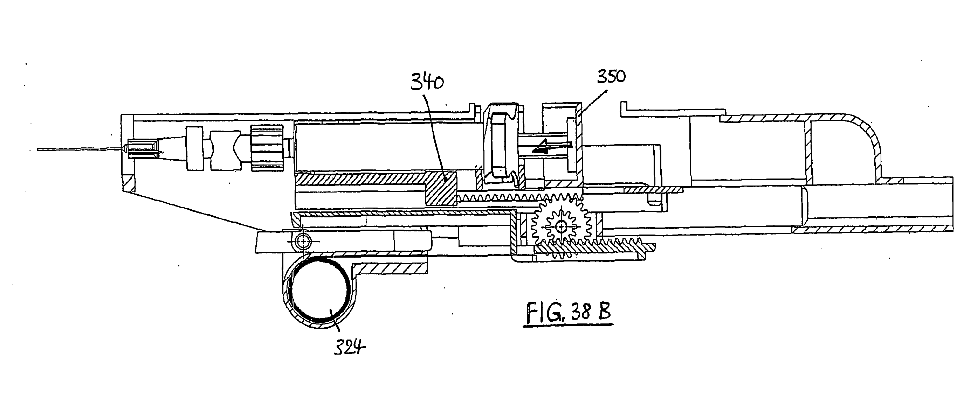

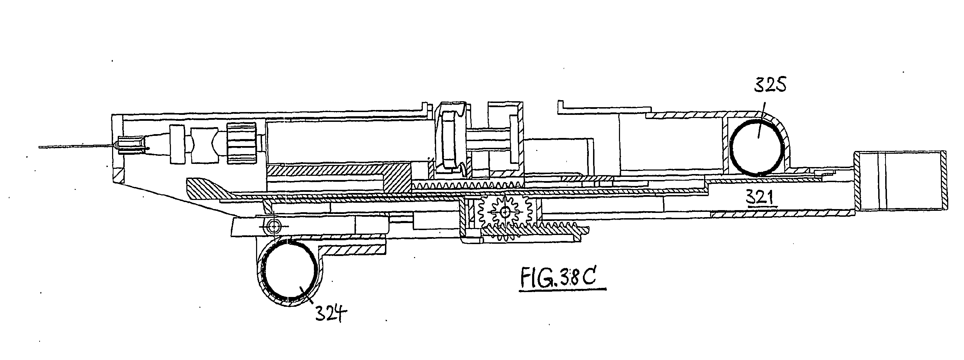

Beim weiteren Vorschub des Vorschubschlittens 323 drückt die Stirnfläche 352 des Stössels 350 über den Flansch 302 und die Kolbenstange 305 den Kolben 304 in die Spritze 300, das Arzneimittel wird injiziert. Die Verriegelungsarme 351A, 351B des Stössels 350 federn an Schubnasen 341A,341B der Spritzenaufnahme 340 vorbei (Injektionshub H2, Figur 38).

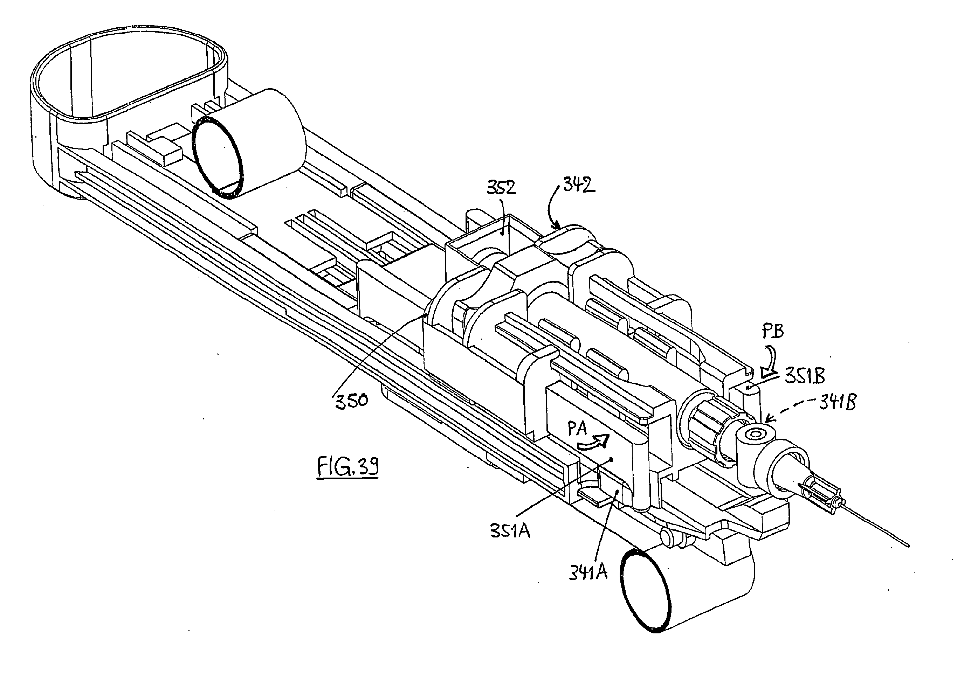

Der Injektionshub H2 ist beendet (Figur 39), wenn der Stössel 350 an die hintere Wandung 342 der Spritzenaufnahme 340 anschlägt und die Rasthaken 351A, 351 B des Stössels 350 hinter den Schubnasen 341A,341B der Spritzenaufnahme 340 eingerastet sind (Pfeile PA,PB). Das Arzneimittel ist jetzt injiziert. Ein Auslenken der Verriegelungsarme 323A, 323B des Vorschubschlittens 323 ist jetzt möglich und der Vorschubschlitten 323 kann mit dem Verfahren zur Durchführung des Rückholhubs H3 beginnen.

Um eine vollständige Injektion des Arzneimittels zu gewährleisten, darf der Spritzenrücklauf erst nach einer gewissen Zeitverzögerung beginnen. Deshalb muß sich der Vorschubschlitten 323 mit seinen Anschlagstangen 328A,328B (Figur 26-28) noch ca.2.5 mm bis zur Anschlagswand 312F des Aufnahmerahmens 312 bewegen.

Hierzu schwenken die Verriegelungsarme 323A, 323B des Vorschubschlittens 323 an den Rasthaken 351C, 351 D des Stössels 350 nach unten aus und die Zahnräder 313A, 313B bewegen den Vorschubschlitten 323 über das Zahnrad 313C in Richtung zur Anschlagswand 312F.

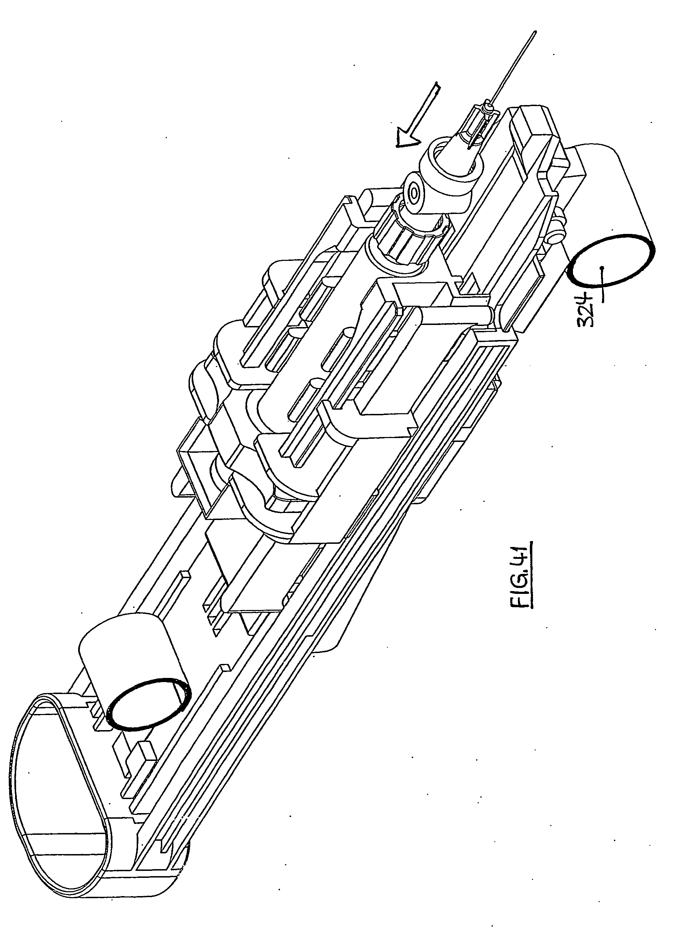

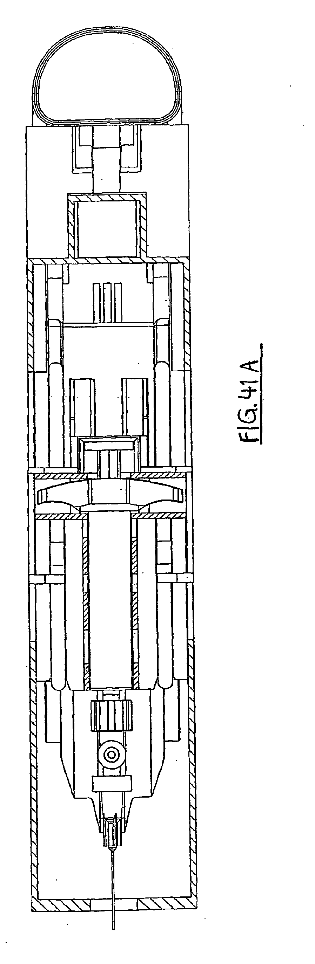

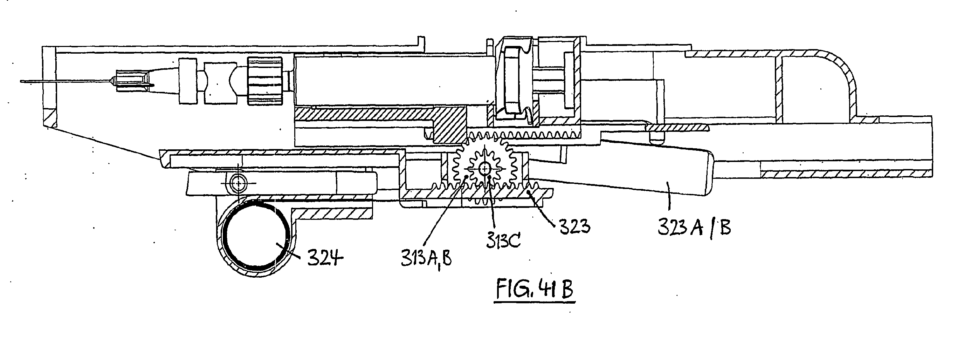

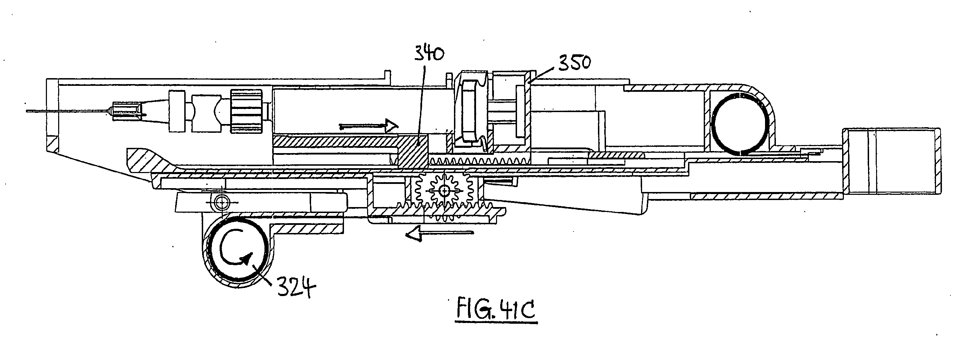

Zum Spritzenrücklauf (Rückholhub H3, Fig. 41) liegt der Vorschubschlitten 323 mit seinen Anschlagstangen 328A,328B an der Anschlagswand 312F des Aufnahmerahmens 312 an. Die Vorschubfeder 324 zieht den Vorschubschlitten 323 weiter. Über das Zahnradgetriebe 328 werden Stössel 350, Spritzenaufnahme 340 und Spritze 300 wieder zurückbewegt.

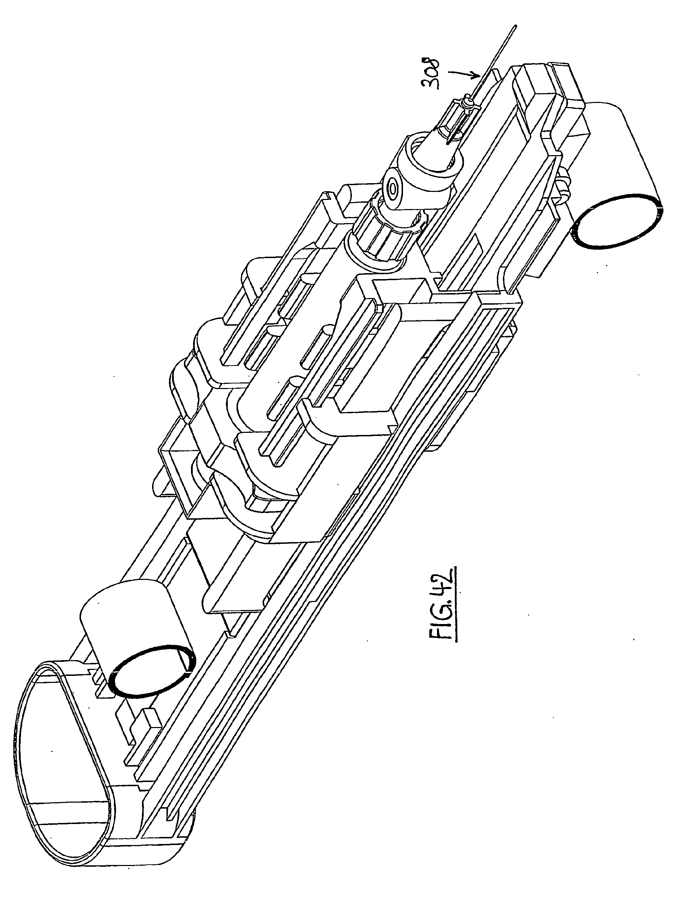

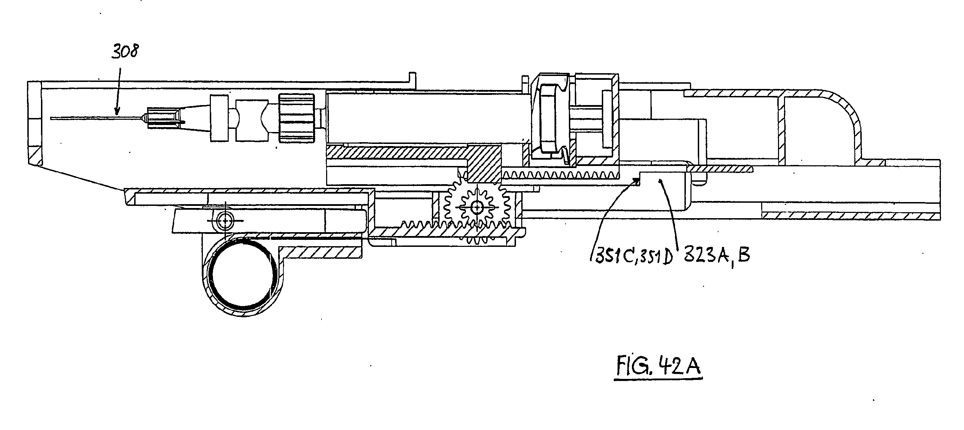

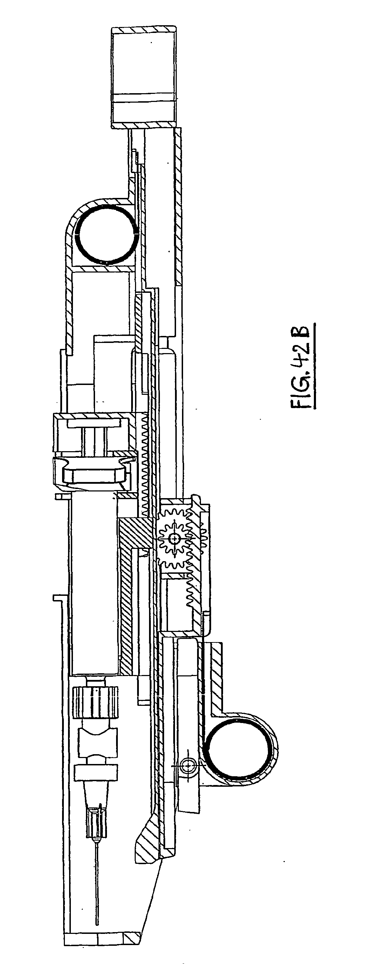

Der Spritzenrücklauf ist beendet Figur 42), wenn die Spritzenaufnahme 340 gegen den Anschlag 312C, 312D des Aufnahmerahmens 312 (Figur 33) gefahren ist. Die Verriegelungsarme 323A, 323B des Vorschubschlittens 323 rasten hinter den Rasthaken 351C, 351D des Stössels 350 wieder ein. Die Nadel 308 ist vollständig aus der Haut gezogen.

Am Ende des Spritzenrückzugs wird durch den Vorschubschlitten 323 der Klingelmechanismus 380 (figur 32) ausgelöst.

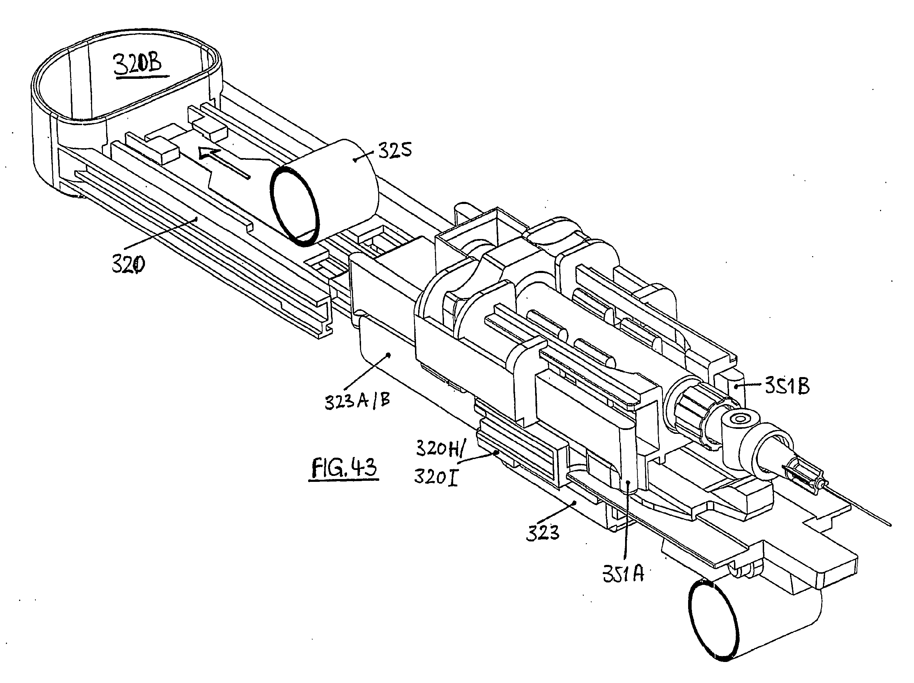

Zum Laden der Injektionsvorrichtung (Figuren 43-47) muß die Ladezugstange 320 an ihrem Griff 320B aus dem Aufnahmerahmen 312 herausgezogen werden. Die Verriegelungsarme 323A, 323B des Vorschubschlittens 323 werden durch Sperrhaken 320H, 320I der Ladezugstange 320 gesperrt. Gleichzeitig werden die Rasthaken 351 A, 351 B des Stössels 350 freigeschaltet und Zugkanten der Ladezugstange 320 treffen auf die Verriegelungsarme 323A, 323B des Vorschubschlittens 323.

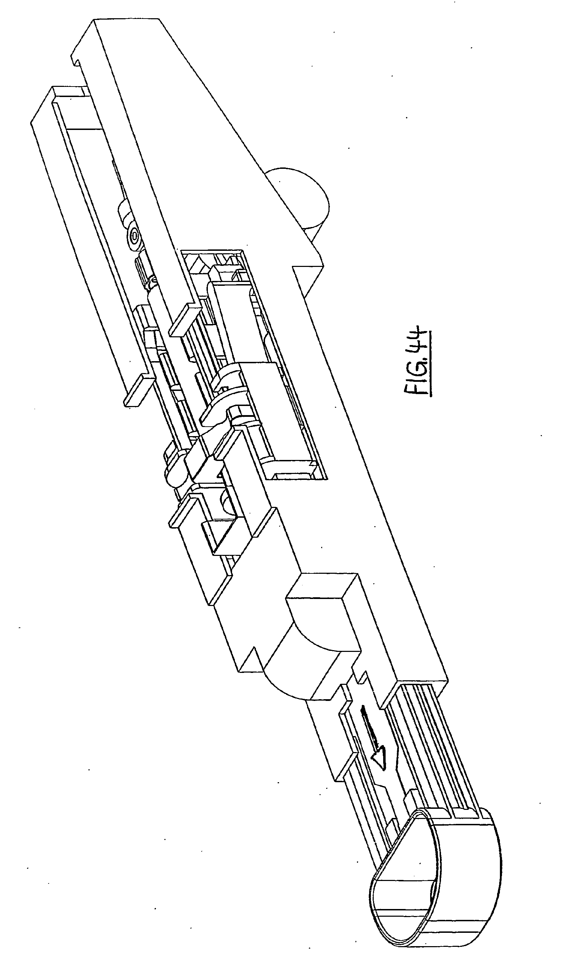

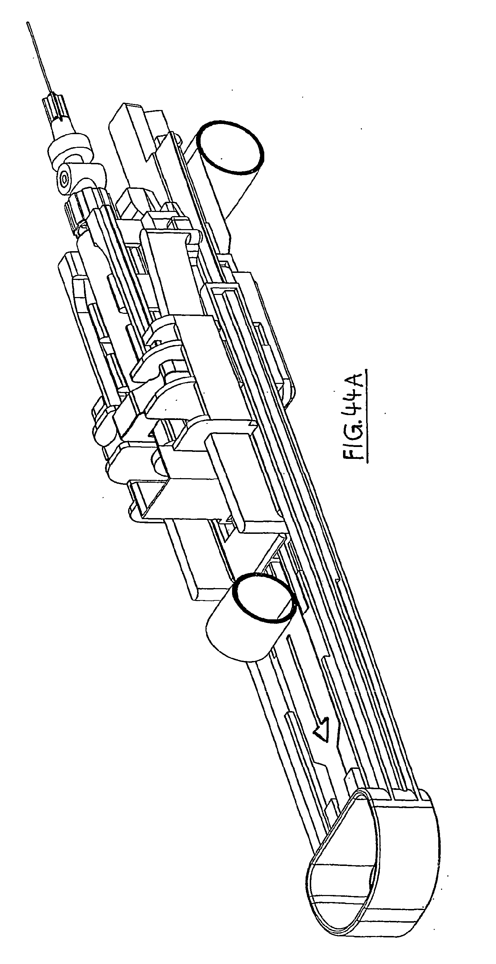

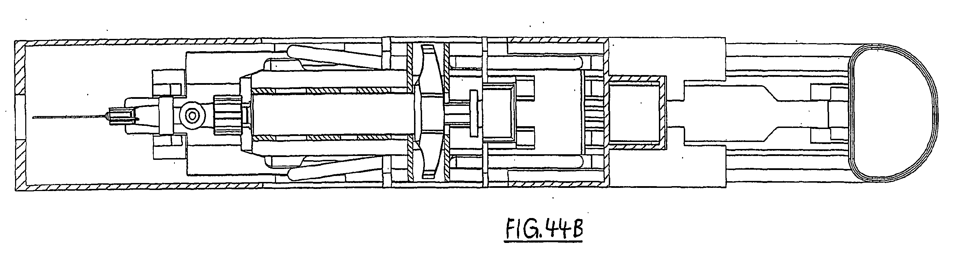

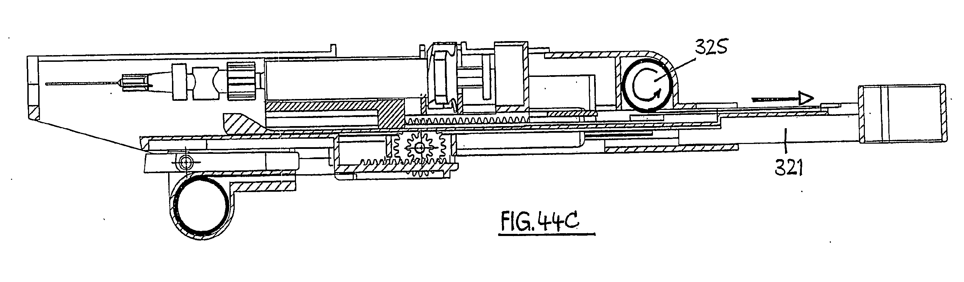

Wird die Ladezugstange 320 weiter herausgezogen (Figur 44), bewegt sich der Injektionsschlitten mit dem Stössel 350 wieder in seine Ausgangsposition zurück. Die Verriegelungsarme 351A, 351 B des Stössels 350 federn an den Schubnasen 341 der Spritzenaufnahme 340 vorbei.

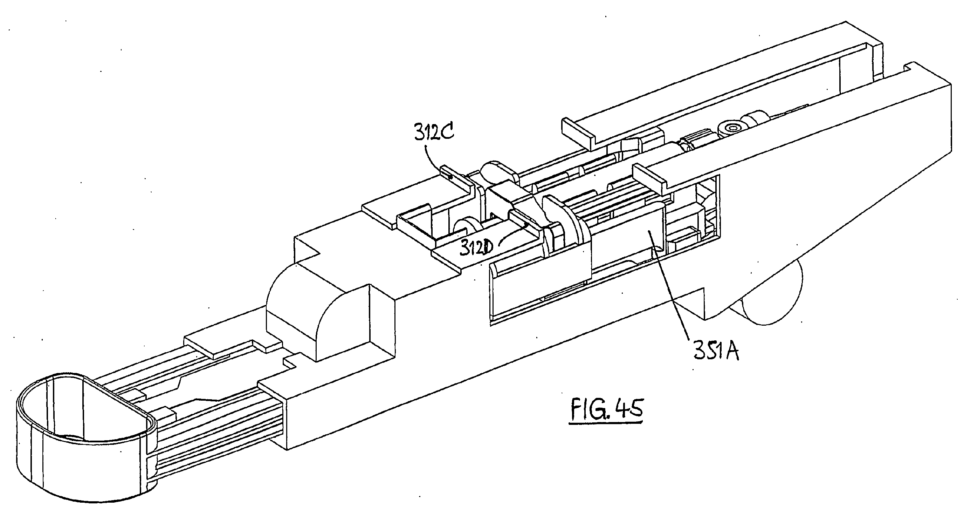

Der Injektionsschlitten befindet sich im weiteren Verlauf des Ladevorgangs (Figur 45) wieder am Anschlag 312C,312D des Aufnahmerahmens 312. Die Verriegelungsarme 351A, 351B des Stössels 350 sind wieder hinter die Schubnasen 341 der Spritzenaufnahme 340 eingerastet. Die Verriegelungsarme 351A, 351 B des Stössels 350 sind durch die Ladezugstange 320 und die Spritzenaufnahme 340 freigeschaltet und können auslenken.

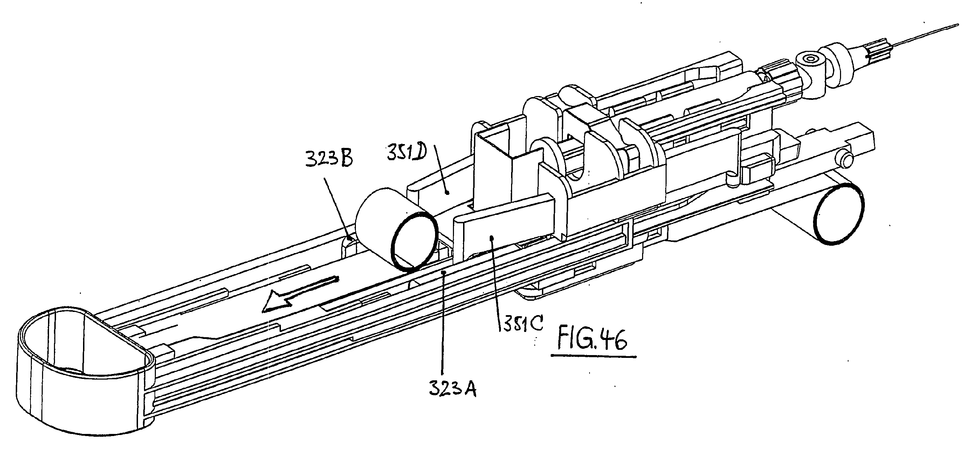

Beim weiteren Herausziehen der Ladezugstange 320 (Figur 46) schwenken die Rasthaken 351C, 351D des Stössels 350 an den Verriegelungsarmen 323A, 323B des Vorschubschlittens 323 nach innen und laufen seitlich an diesen vorbei. Der Vorschubschlitten 323 mit dem Zahnradgetriebe 328 bewegen sich in Ihre Startposition zurück.

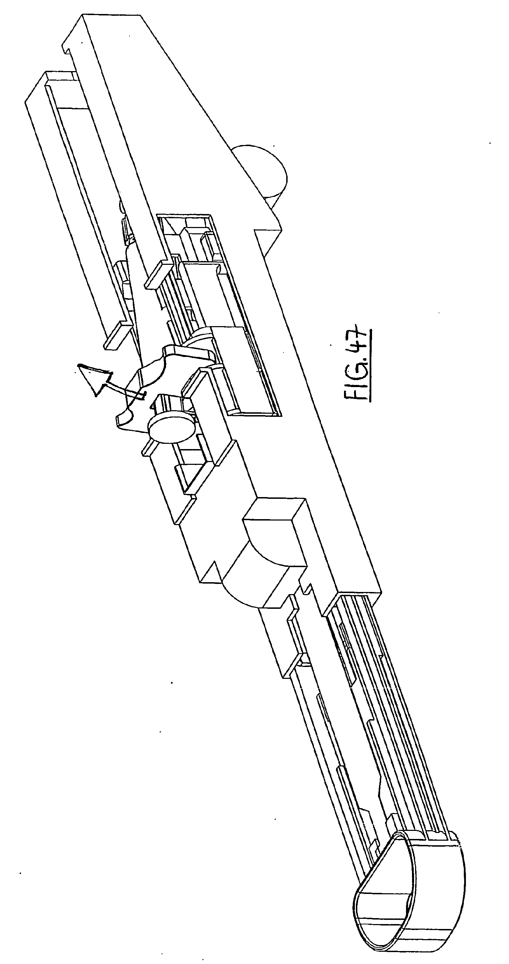

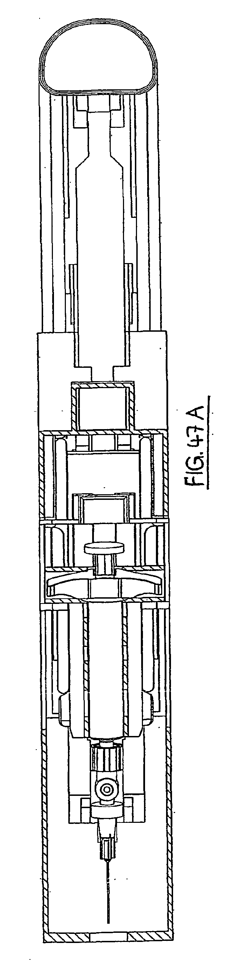

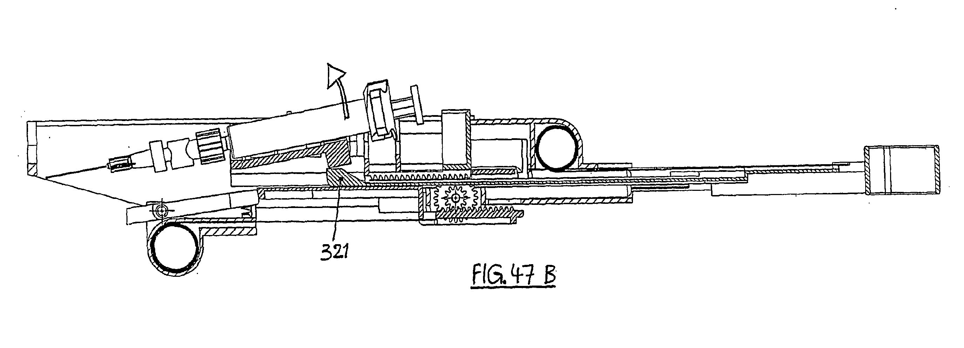

Am Ende des Ladevorgangs (Figur 47) rasten die Verriegelungsarme 351A, 351 B des Stössels 350 hinter den Verriegelungsarmen 351A, 351 B des Vorschubschlittens 323 wieder ein. Vorschubschlitten 323 mit Zahnradgetriebe 328 befinden sich wieder in der Startposition.

Am Ende der Ladezugstange 320 befindet sich eine Drucknase 321E, die einen Auswurfhaken 343 (Figur 36C) an der Spritzenaufnahme 340 betätigt und die Spritze 300 zur besseren Entnahme nach oben kippt.

Nach dem Loslassen des Griffes 320B der Ladezugstange 320 wird diese durch die Rückzugskraft der Rückzugsfeder 325 wieder in Ihre Ausgangsposition zurückgezogen.

Der Vorschubschlitten 323 rastet wieder hinter dem Auslöseschwenkhebel 374 ein und ist durch die Rückzugskraft der Vorschubfeder 324 wieder gespannt. Am Ende des Spritzenrückzuges wird durch den Vorschubschlitten 323 der Klingelmechanismus 380 (Figur 32) ausgelöst.

### Viertes Ausführungsbeispiel

Der Grundaufbau des Injektomaten entspricht in seinen Hauptbestandteilen dem des dritten Ausführungsbeispiel, so dass im folgenden nur die wesentlichen Unterschiede in Aufbau und Funktion dargestellt sind.

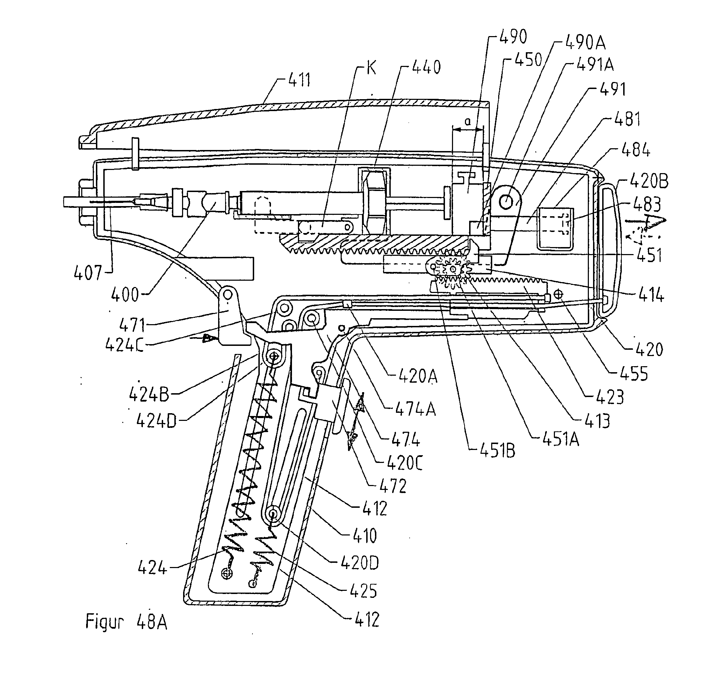

Figur 48A zeigt die wesentlichen Elemente des vierten Ausführungsbeispiels:
Die Spritze 400 ist in die Spritzenaufnahme 440 eingelegt. Zwischen dem Stössel 450 und der Spritzenaufnahme 440 ist ein Volumenadapter 490 eingelegt und verrastet, durch den der Injektionshub H2 verkürzt werden kann, indem der Abstand des Endes des Spritzenkolbens von der Innenwand der Spritzenaufnahme verkürzt wird. Entsprechend des gewünschten Injektionsvolumens (z.B. 0,5, 0,75 oder 1,0 ml) wird ein geeigneter Volumenadapter 490 in den Stössel 450 eingeschoben. Die jeweiligen Volumenadapter 490 unterscheiden sich durch den Abstand a und die Lage einer Steuerrippe 490A, die sich an dem jeweiligen Volumenadapter befindet. Die Steuerrippe 490A wirkt mit einem Volumensteuerhebel 491 zusammen.

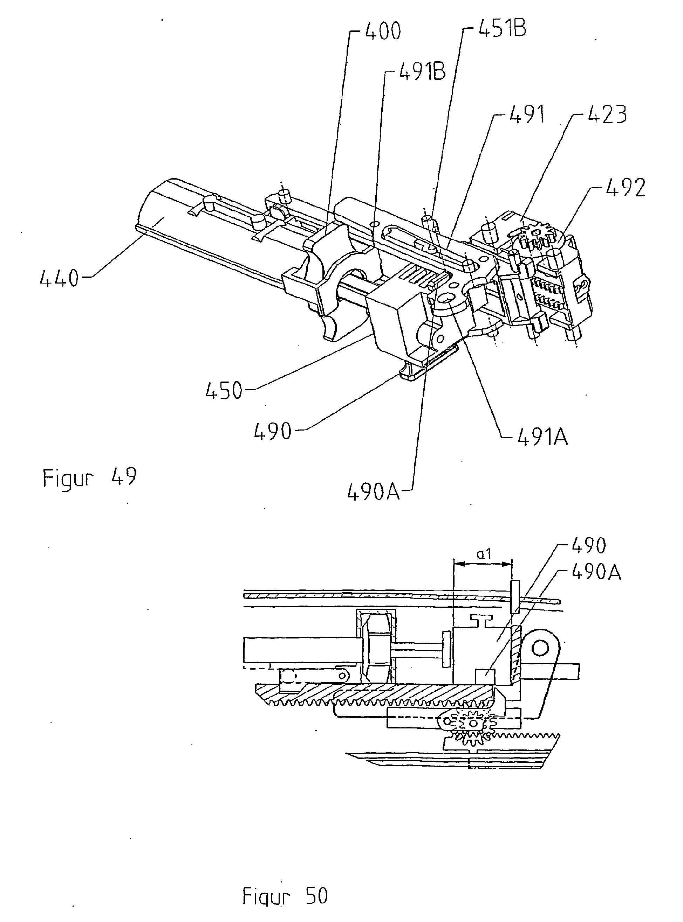

Figur 50 zeigt einen grösseren Volumenadapter für ein kleineres Injektionsvolumen als in Figur 48A. (a1 > a, Lage der Steuerrippe 490A verändert sich).

Zur Durchführung der Hube ist eine Anordnung von Seilzug 424B, Ladezugseil 420, Zugfeder 424 und Rückzugsfeder 425 vorgesehen, wobei die Zugfeder 424 die Vorschubkraft erzeugt und über eine Anordnung nach Art eines Flaschenzugs über eine Umlenkrolle 424 D über den Seilzug 424 B mit einer entsprechend untersetzen Zugkraft am Vorschubschlitten 423 angreift. Das Ladezugseil 420 läuft ebenfalls über eine Umlenkrolle 420 D, die mit der Rückzugsfeder 425 verbunden ist, zu einem Griff 420B an der Stirnseite des Gehäuses 410 und nimmt über einen Mitnehmer 420A den Vorschubschlitten mit.

Eine weitere, wesentliche Weiterbildung des Injektomaten besteht in der Konzeption dieser Bauteile derart, dass nach Durchführung des Injektionshubs H2 eine Verweilzeit TV einstellbar ist , nach deren Ablauf erst der Rückholhub H3 beginnt. Diese Verweilzeit hat den Vorteil, dass sich der Druck im subkutanen Gewebe, der durch das Einspritzen des Arzneimittels erzeugt wird, abbauen kann, bevor die Nadel heraus gezogen wird, wodurch das Eindringen von Arzneimittel in den Einstechkanal der Nadel weitgehend vermieden wird.

Konstruktiv wird diese Wirkung dadurch erreicht, dass der Vorschubschlitten 423 und das Gehäuse 414 mit dem Doppelzahnrad 413 während der Verweilzeit TV zwar sich weiter bewegen, aber ohne weitere Ankopplung des Stössels 450, und die Initiierung des Rückholhubs H3 mit der entsprechenden Ankopplung der Spritzenaufnahme 440 aber erst nach einem die Verweildauer TV bestimmenden Leerhub H0 des Vorschubschlittens 423 erfolgt.

Je nachdem, welcher Volumenadapter 490 eingesetzt wird, ändert sich der Umschaltpunkt vom Injektionshub H2 auf den Leerhub H0.

Eine weitere Ausgestaltung besteht in der Anordnung eines Dämpfungsgliedes 492 (Figur 49). Ein dersrtiges Dämpfungsglied ist dem Vorschubschlitten zugeordnet und dämpft dessen Bewegung während des Vorschubs, um durch eine langsamere Bewegung des Stössels 450 längere Injektionszeiten zu erzielen.

Weiter ergänzende Bauteile werden in der nun folgendenBeschreibung des Funktionsablaufs erläutert:
Im Ausgangszustand der Injektionsvorrichtung ist die Vorschubfeder (Zugfeder) 424 gespannt und wirkt über die Zugrolle 424D auf das Zugseil 424B. Ein Ende des Zugseiles 424B ist am Aufnahmerahmen 412 und das andere Ende am Vorschubschlitten 423 befestigt, wobei eine Umlenkung des Zugseiles 424B über die Umlenkrolle 424C erfolgt. Aufgrund der Wirkungsweise eines einfachen Flaschenzuges wirkt auf den Vorschubschlitten 423 die halbe Kraft der Vorschubfeder 424. Um den Federweg zu minimieren, bzw. die Weg/Kraft Kennlinie bezogen auf den Vorschubschlitten 423 dem individuellen Anwendungsfall anzupassen, ist auch ein doppelter Flaschenzug, kombiniert mit einer oder mehrerer Federn möglich.

Die Rückzugsfeder 425 (Zugfeder) ist bis auf die Vorspannung entspannt, wirkt über die Rolle 420D mit ihrer halben Kraft auf das Ladezugseil 420, welches ebenfalls nach Art eines Flaschenzuges mit einem Ende am Aufnahmerahmen 412 und mit seinem anderen Ende am Griff 420 B befestigt ist. Das Ladezugseil wird durch den Vorschubschlitten 423 geführt, ist jedoch mit diesem nicht verbunden. Auf dem Ladzugseil 420 ist ein Mitnehmer 420A befestigt, dessen Außendurchmesser größer ist als die Bohrung im Vorschubschlitten 423, durch die das Ladezugseil 420 geführt wird.

Der Vorschubschlitten 423 ist somit mit der halben Kraft der Vorschubfeder 424 beaufschlagt, er bleibt in seiner Position, da er durch einen Auslöseschwenkhebel 474 mit Drehpunkt 474 A abgestützt wird.

Der mechanische Ablauf wird durch Betätigen eines tastenartigen Schaltelements 471 ausgelöst, das über eine Schräge den Auslöseschwenkhebel 474 um den Drehpunkt 474 A verschwenkt und dadurch den Vorschubschlitten 423 freigibt.

Der Auslöseschwenkhebel 474 kann jedoch nur geschwenkt werden, wenn zuvor eine Schiebesicherung 472 in Richtung des Pfeiles A (Freigabeposition) geschoben wurde.

Nach der Auslösung durch das Schaltelement 471 ergibt sich eine starre Verbindung des Vorschubschlittens 423 mit dem Stössel 450, da die Verzahnung des Vorschubschlittens 423 mit dem kleineren Zahnrad des Zahnradpaares 413 und das größere Zahnrad mit der Verzahnung des Stössels 450 kämmt, das Zahnradpaar im Gehäuse 414 gelagert ist, und ein Mitnahmehebel 451, der ebenfalls im Gehäuse 414 drehbar gelagert ist, formschlüssig in den Stössel 450 eingreift.

Durch ein Kopplungselement K, hier als Klinke dargestellt, die den Stössel 450 mit der Spritzenaufnahme 440 verbindet, sind die Spritzenaufnahme 440 und der Stössel 450 so gekoppelt, dass sie zunächst in einer gleichförmigen Bewegung den Einstechhub (Hub 1) ausführen.

Der Mitnahmehebel 451 ist in einem Stift 451A drehbar gelagert. Durch den Abstand zwischen dem Stift 451 A und dem Kraftangriffspunkt am Stössel 450 ergibt sich ein rechtsdrehendes Drehmoment, sobald sich der Vorschubschlitten 423 bewegt und die Vorschubkraft über die Zahnräder und den Mitnahmehebel 451 auf den Stössel 450 übertragen wird. Eine Drehung des Mitnahmehebels 451 wird jedoch zu diesem Zeitpunkt durch einen Nocken 451 B verhindert, der an einem Steuerhebel 491 anliegt.

Der Steuerhebel 491 ist an seiner Lagerstelle 491A drehbar am Aufnahmerahmen 412 gelagert, kann sich jedoch nicht drehen, da er an der Steuerrippe 490A des Volumenadapters 490 ansteht.

Daher führen die Spritzenaufnahme 440 und der Stössel 450 gemeinsam den Hub 1 (Einstechhub) aus. Dabei gleitet die Steuerrippe 490A auf dem Steuerhebel 491 und verhindert dessen Ausschwenken und damit auch eine Drehung des Mitnahmehebels 451.

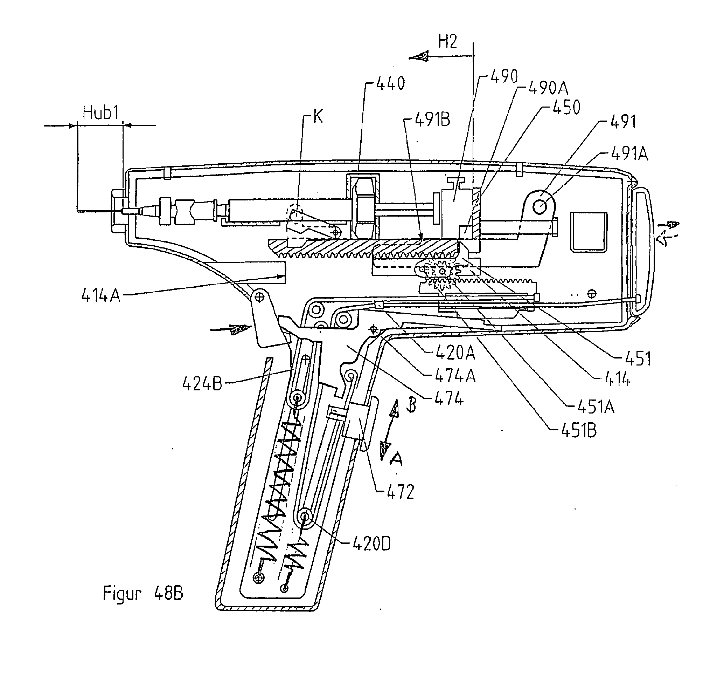

Nach dem Einstechhub 1 wird die Verbindung von Spritzenaufnahme 440 und Stössel 450 durch ein Ausschwenken des Kopplungselementes K gelöst (Figur 48B). Die Spritzenaufnahme 440 verbleibt in ihrer Position, der Stössel 450 wird weiterbewegt, der Einstechhub H2 und die Injektion des Arzneimittels beginnt.

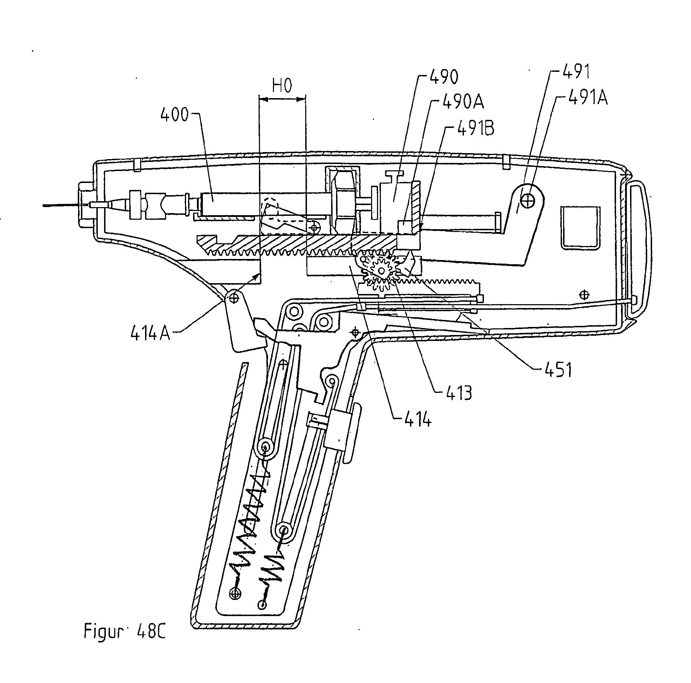

Da sich der Volumenadapter 490 und damit die Steuerrippe 490A bei der Injektion vom Lagerpunkt des Stifts 491 A des Steuerhebels 490 wegbewegt, können sich der Steuerhebel 490 und damit der Mitnahmehebel 451 so lange nicht um ihre Lagerstellen drehen, bis die Steuerrippe 490A die Schräge 491 B erreicht. Sobald diese erreicht ist, kann der Steuerhebel 491 durch einen Nocken 451 B angehoben werden, der Mitnahmehebel 451 dreht sich um seine Lagerung 451A und der Formschluss mit dem Stössel 450 wird aufgegeben. Der Injektionshub H2 ist beendet (Figur 48C).

Wird ein Volumenadapter 490 für ein kleineres Injektionsvolumen eingesetzt, vergrößert dieser den Abstand zwischen der Rückwand des Stössels 450 und der Kolbenstange. Die Steuerrippe 490A sitzt dann näher bei der Schräge 491B. d.h., der Injektionshub H2 wird kleiner, da die Steuerrippe 490A die Schräge 491 B nach einem kürzeren Weg erreicht.

Wie in Figur 48C dargestellt, hat das Gehäuse 414 mit dem Doppelzahnrad 413 zum Zeitpunkt der Freigabe der Drehung des Mitnahmehebels 451 einen Anschlag 414A noch nicht erreicht. Deshalb wälzt sich das Zahnradpaar 413 lediglich auf den beiden Verzahnungen ab, bis der Anschlag 414A erreicht ist.

Dieser dadurch erzeugte Leerhub H0 sorgt dafür, dass der Nadelrückzug nicht sofort nach dem Einstechhub H2 erfolgt, sondern um die Verweilzeit TV zeitlich verzögert.

Erst, wenn der Anschlag 414A erreicht ist, wird der Stössel 450 in die entgegengesetzte Richtung bewegt, wobei eine Übersetzung entsprechend der Teilkreise der beiden Zahnräder erfolgt. Nach einem Weg, der dem Betrag des Einstechhubs H1 entspricht, koppelt die Spritzenaufnahme 440 hinzu, dadurch wird die Nadel automatisch zurückgezogen und der Rückholhub H3 ist beendet.

Vor einer erneuten Injektion muss die Vorschubfeder 424 gespannt werden:
Gehäuse 414 und Vorschubschlitten 423 befinden sich in ihrer Endlage, die Vorschubfeder 424 ist bis auf ihre Vorspannung entspannt. Der Mitnehmer 420A liegt an der Wand des Vorschubschlittens 423 an. Wird nun am Griff 420B gezogen, transportiert der fest auf dem Ladezugseil 420 befestigte Mitnehmer 420A den Vorschubschlitten 423 in seine Ausgangslage, der Auslöseschwenkhebel 474 schwenkt vor den Vorschubschlitten 423 und fixiert diesen. Während des Rückzugs des Vorschubschlittens 423 wird mit Hilfe des Zugseiles 424B, welches fest mit dem Vorschubschlitten 423 verbunden ist, die Vorschubfeder 424 gespannt. Gleichzeitig wird die Rückzugsfeder 425 mit Hilfe der Zugrolle 420D gespannt, wobei die im Aufnahmerahmen 412 geführte Zugrolle 420D die Schiebesicherung 472 in Richtung des Pfeils B (Sicherungsposition) bewegt.

Sobald der Griff 420B losgelassen wird, zieht sich das Ladezugseil 420 wieder auf seine ursprüngliche Position ein.

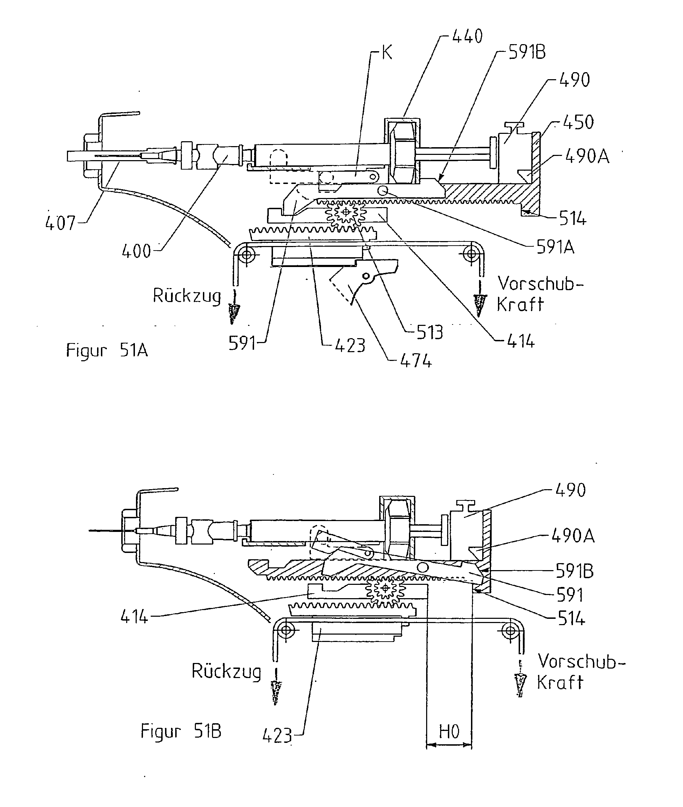

Die Figuren 51 - 53 zeigen Varianten der Antriebsankopplung, mit denen der Funktionsablauf Einstechhub H1- Injektionshub H2 - Leerhub H0 (Verweilzeit TV) - Rückholhub H3 ebenfalls erreicht werden kann.

Figur 51A zeigt einen Aufbau, in dem die Spritze 400, die Spritzenaufnahme 440, der Stössel 450, der Volumenadapter 490 und das Kopplungselement K die gleiche Aufgabe haben, wie sie oben zum vierten Ausführungsbeispiel beschrieben sind.

In einem Gehäuse 414 ist wiederum ein Zahnradpaar 513 gelagert, dessen größeres Zahnrad im Gegensatz zum vierten Ausführungsbeispiel mit dem Vorschubschlitten 423 kämmt und im Stössel 450 in einer Nut frei läuft, das kleinere Zahnrad mit der Verzahnung auf dem Stössel 450 kämmt.

Der Vorschubschlitten 423 ist durch die Kraft einer Vorschubfeder so belastet, dass er sich nach rechts bewegen möchte, jedoch durch den Auslöseschwenkhebel 474 daran gehindert wird.

Der Steuerhebel 591 ist im Rahmen 412 mit einem Drehlager 591 A gelagert und greift in der Ausgangslage formschlüssig in das Gehäuse 414 ein.

Sobald der Vorschubschlitten 423 durch den Auslöseschwenkhebel 474 freigegeben wird, bewegt sich der Vorschubschlitten nach rechts. Da das Gehäuse 414 durch den Steuerhebel ortsfest fixiert ist, dreht sich das Zahnradpaar, Stössel 450 und Spritzenaufnahme 440 bewegen sich gemeinsam nach links. Es erfolgt eine Untersetzung (Weg des Stössels < Weg Vorschubschlitten).

Nach dem gemeinsamen Einstechhub H1 wird der Stössel von der Spritzenaufnahme entkoppelt und es erfolgt der Injektionshub H2.

Sobald die Steuerrippe 490A des Volumenadapters 490 die Schräge 591 B des Steuerhebels 591 A erreicht, wird dieser geschwenkt und der Formschluss mit dem Gehäuse 414 aufgehoben (Figur 51 B).

Zu diesem Zeitpunkt hat das Gehäuse den Anschlag 514 am Stössel 450 noch nicht erreicht, deshalb erfolgt der Leerhub H0 (Verweilzeit TV) bis zum Erreichen des Anschlages 514.

Ist der Anschlag 514 erreicht, wird der Stössel 450 gleichförmig mit der Bewegung des Vorschubschlittens 423 nach rechts mitgenommen. Nach einem Weg, der im Betrag dem Einstechhub H1 entspricht, koppelt die Spritzenaufnahme 440 ein und der Nadelrückzug während des Rückholhubs H3 erfolgt.

Der Vorteil dieses Prinzips liegt darin, dass beim größten Kraftbedarf, dem Injektionshub H2, eine Untersetzung wirkt, und deshalb die Vorschubkraft kleiner gewählt werden kann, und dadurch auch die manuell aufzubringende Kraft zum Spannen der Federn kleiner wird.

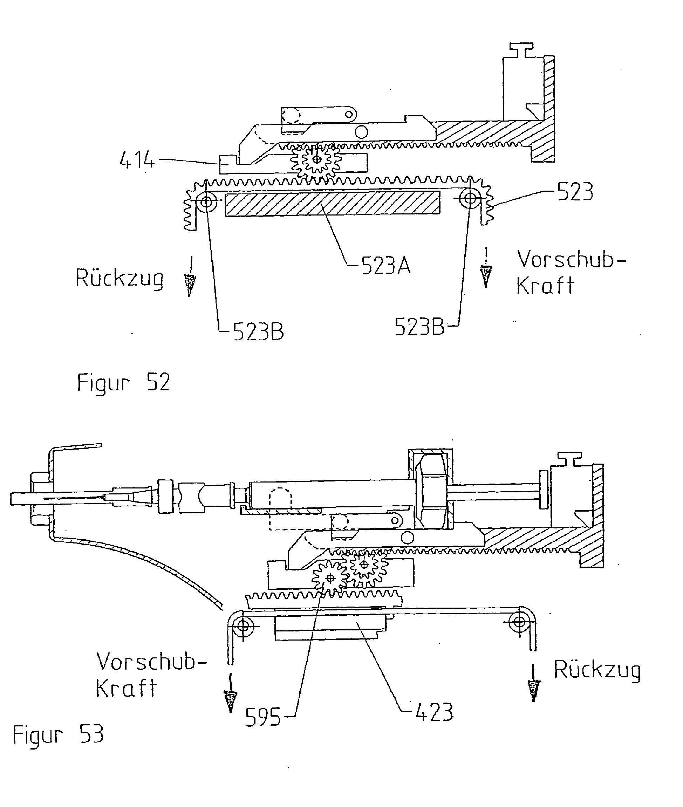

Hierbei muss der Vorschubschlitten 423 einen längeren Weg zurücklegen. Aus Platzgründen kann es deshalb vorteilhaft sein, den Antrieb mit Hilfe eines Zahnriemens 523, der auf einer Unterlage 523A gleitet und durch Rollen 523B umgelenkt wird, zu gestalten (Figur 52).

Zur Definition der Bewegungsrichtung des Vorschubschlittens 423 bzw. des Zahnriemens 523 kann ein Zwischenrad 595, wie in Figur 53 dargestellt, zwischen geschaltet werden.

Es versteht sich von selbst, dass der geschilderte mechanische Aufbau zumindest teilweise auch mit Hilfe von elektrischen/elektronischen Bauteilen durchgeführt oder ergänzt werden kann, z. B. durch Schrittschaltmotore zur Erzeugung der Hube, Sensoren zur Erfassung der Positionen der Funktionsbauteile, elektronische Signaleinrichtungen u.ä.

## Patentansprüche

1. Injektionsvorrichtung für eine Spritze, mit Spritzenkörper , Kanüle mit Nadel, Kolben mit Kolbenstange, und mit mindestens einem Betätigungselement (120,220,320) zur Umsetzung der vom Patienten manuell aufzubringenden Betätigungsarbeit in eine Verschiebung des Spritzenkörpers (101,201,301) während eines Einstechhubs (H1) und eines Rückholhubes (H3), sowie in eine Verschiebung der Kolbenstange während eines Injektionshubs (H2), mit einer Führungseinrichtung zur Halterung des Spritzenkörpers (101,201,301) und einem gegen diese verschiebbaren Stössel (150,250,350) zur Verschiebung der Kolbenstange, wobei die Betätigungsarbeit mittels einer einzigen, gerichteten linearen Bewegung des Betätigungselements (120,220,320) in den Einstechhub (H1), den Injektionshub (H2) und den Rückholhub (H3) derart umgesetzt wird, dass die Führungseinrichtung und der Stössel (150,250,350) vom Betätigungselement beim Einstechhub (H1) gemeinsam beaufschlagt werden, und beim Injektionshub (H2) lediglich der Stössel (150,250,350) beaufschlagt wird,
**dadurch gekennzeichnet, dass** die Führungseinrichtung eine verschiebbare Spritzenaufnahme (140,240,340), in der die Spritze (100,200,300,400) fixiert ist, beinhaltet, die mit dem Stössel (150,250,350) lösbar gekoppelt ist und Teil eines Injektionsschlittens ist, den zur Durchführung eines Rückholhubs (H3), der betragsmässig im Wesentlichen dem Einstechhub (H1) entspricht, das Betätigungselement (120,220,320,420) unter Zwischenschaltung eines weiteren Schlittens (114A,260,323,423) mittels Rast- und Koppelelementen positions- und richtungsdefiniert beaufschlagt.

2. Injektionsvorrichtung nach Anspruch 1, **dadurch gekennzeichnet, dass** das Betätigungselement eine in einem Gehäuse (110,210) parallel zum Injektionsschlitten geführte Schubstange (120,220) ist, durch deren Einschub in das Gehäuse (110,210) auch die Bauteile zur Erzeugung des Rückholhubes (H3) betätigt und/oder aktiviert werden.

3. Injektionsvorrichtung nach Anspruch 2, **dadurch gekennzeichnet, dass** die Bauteile zur Erzeugung des Rückholhubes (H3) mindestens ein in den Injektionsschlitten (140,150) und die Schubstange (120) eingreifendes Zahnrad (113) beinhaltet, das in einem im Gehäuse (110) verschiebbaren Schlitten (114A) gelagert ist, und dass das Zahnrad (113) mit einem Sperrelement zusammenwirkt, das das Zahnrad (113) blockiert, wenn Einstechhub (H1) und Injektionshub (H2) ausgeführt werden, und das das Zahnrad (113) danach freigibt, wodurch die Linearbewegung der Schubstange (120) in den gegengerichteten Rückholhub (H3) des Injektionsschlittens (140,150) umgesetzt wird.

4. Injektionsvorrichtung nach Anspruch 3, **dadurch gekennzeichnet, dass** mindestens zwei Zahnräder (113A,113B) zur Erzeugung einer Übersetzung der Linearbewegung der Schubstange (120) in den Rückholhub (H3) im gemeinsamen Schlitten (114A) vorgesehen sind.

5. Injektionsvorrichtung nach Anspruch 3, **dadurch gekennzeichnet, dass** das Sperrelement eine linear am Schlitten (114A) verschiebbare Sperrklinke (114) ist, die in der Sperrposition in die Zähne des Zahnrads (113) eingreift.

6. Injektionsvorrichtung nach Anspruch 3, **dadurch gekennzeichnet, dass** das Sperrelement ein Schwenkhebel (114B) ist, der in der Sperrposition in die Zahnung der Schubstange (120) eingreift.

7. Injektionsvorrichtung nach Anspruch 2, **dadurch gekennzeichnet, dass** die Kopplung zwischen Spritzenaufnahme (140) und Stössel (150) von zwei Kulissensteinen (145A,145B) bewirkt wird, die in einen lösbaren Formschluss zwischen Spritzenaufnahme (140) und Gehäuse (110), und zwischen Spritzenaufnahme (140) und Stössel (140) bringbar sind.

8. Injektionsvorrichtung nach Anspruch 2, **dadurch gekennzeichnet, dass** die Kopplung zwischen Spritzenaufnahme (140) und Stössel (150) von einem weiteren, ebenfalls im Schlitten (114A) gehaltenen Zahnrad (113C) bewirkt wird, das während des Einstechhubes (H1) blockiert wird.

9. Injektionsvorrichtung nach Anspruch 2, **dadurch gekennzeichnet, dass** die Bauteile zur Erzeugung des Rückholhubs (H3) mindestens ein Federelement (261A,261B) als Energiespeicher beinhalten, das vor Beginn der Injektion von der Schubstange (220) vorgespannt (Spannhub) und nach dem Injektionshub (H2) zur impulsartigen Beaufschlagung eines mit dem Injektionsschlitten lösbar verbundenen und an der Spritzenaufnahme (240) anliegenden Rückholschlittens (260) zur Erzeugung des Rückholhubes (H3) freigeschaltet wird.

10. Injektionsvorrichtung nach Anspruch 2, **dadurch gekennzeichnet, dass** in der Schubstange (220) ein drehbar gelagerter Steuerhebel (221) vorgesehen ist, dessen eine Stirnseite in den Injektionsschlitten (240,250) eingreift, wenn der Spannhub abgeschlossen ist.

11. Injektionsvorrichtung nach Anspruch 10, **dadurch gekennzeichnet, dass** der Steuerhebel (221) durch Drehung um einen Steuerwinkel auch die Lösung der Kopplung zwischen Spritzenaufnahme (240) und Stössel (250) beim Übergang vom Einstechhub (H1) zum Injektionshub (H2) bewirkt.

12. Injektionsvorrichtung nach Anspruch 9, **dadurch gekennzeichnet, dass** der Rückholschlitten (260) zangenförmige Rastelemente (262A,262B) aufweist, die nach dem Injektionshub (H2) in Ausnehmungen (226A,226B) der Schubstange (220) eintauchen und den Rückholhub (H3) freigeben.

13. Injektionsvorrichtung nach Anspruch 1, **dadurch gekennzeichnet, dass** das Betätigungselement eine Ladezugstange (320) beinhaltet, die beim Herausziehen aus dem Gehäuse (310) mindestens eine Vorschubfeder (324) als Energiespeicher vorspannt, sowie einen Auslösemechanismus (370), der nach Aktivierung den von der Vorschubfeder (324) beaufschlagten Injektionsschlitten (340,350) über einen Vorschubschlitten (323) zur automatischen Durchführung von Einstechhub (H1), Injektionshub (H2) und Rückholhub (H3) freigibt.

14. Injektionsvorrichtung nach Anspruch 13, **dadurch gekennzeichnet, dass** die Ladezugstange (320) nach dem Herausziehen aus dem Gehäuse (310) mindestens eine Rückzugsfeder (325) für den automatischen Rückzug der Ladezugstange (320) als Energiespeicher vorspannt.

15. Injektionsvorrichtung nach Anspruch 13, **dadurch gekennzeichnet, dass** die Vorschubfeder (324) und die Rückzugfedern (325) Rollfedem sind.

16. Injektionsvorrichtung nach Anspruch 14, **dadurch gekennzeichnet, dass** der Auslösemechanismus (370) mit mindestens einem Sicherheitselement (371) gekoppelt ist, das die Auslösung insbesondere nur dann gestattet, wenn die Injektionsvorrichtung auf der Einstichstelle aufgesetzt ist.

17. Injektionsvorrichtung nach Anspruch 13, **dadurch gekennzeichnet, dass** Ladezugstange (320), Vorschubfedern (324,325), Injektionsschlitten (340,350) und Vorschubschlitten (323) in einem Aufnahmerahmen (312) parallel zueinander verschiebbar gehalten sind.

18. Injektionsvorrichtung nach einem der vorhergehenden Ansprüche, **dadurch gekennzeichnet, dass** zur Steuerung der Abläufe, insbesondere der Aufeinanderfolge von Einstechhub (H1), Injektionshub (H2) und Rückholhub (H3) miteinander in/außer Form/Kraftschluss bringbare Steuerelemente, insbesondere am Betätigungselement (120,220,320), an der Spritzenaufnahme (140,240,340), am Stössel (150,250,350) und am Gehäuse (110,210) oder Aufnahmerahmen (312) vorgesehen sind.

19. Injektionsvorrichtung nach Anspruch 18, **dadurch gekennzeichnet, dass** die Steuerelemente elastische Abschnitte, Rastnocken, Aufgleitebenen und Aussparungen beinhalten.

20. Injektionsvorrichtung nach Anspruch 13, **dadurch gekennzeichnet, dass** zur Vorspannung der Vorschubfeder (424) die Ladezugstange durch ein Ladezugseil (420), dessen eines Ende einen Griff (420B) an einer Stirnseite des Gehäuses (410) aufweist, ersetzt ist, das einen mit der Vorschubfeder (424) verbundenen Mitnehmer (420A) aufweist, der am Vorschubschlitten (423) beim Herausziehen des Griffs (420B) angreift.

21. Injektionsvorrichtung nach Anspruch 14 und 20, **dadurch gekennzeichnet, dass** die Vorspannung der Rückzugsfeder (425) ebenfalls durch den Griff (420B) und das Ladezugseil (420) erfolgt, wodurch das Ladezugseil (420) in das Gehäuse (410) bis zum Anschlag des Griffs (420B) am Gehäuse (410) eingezogen wird.

22. Injektionsvorrichtung nach Anspruch 20 und 21, **dadurch gekennzeichnet, dass** Vorschubfeder (424) und Rückzugsfeder (425) als Spiralfedern ausgebildet sind, deren eines Ende in einem im Gehäuse (410) gehaltenen Rahmen (412) befestigt ist, und deren anderes Ende unmittelbar oder über den Mitnehmer (420A) mit dem Ladezugseil (420) verbunden ist.

23. Injektionsvorrichtung nach Anspruch 22, **dadurch gekennzeichnet, dass** das andere Ende des Ladezugseils (420) mit einem im Gehäuse gehaltenen Aufnahmerahmen (412) verbunden ist und über mindestens eine Zugrolle (420D) geführt ist, an deren Achse das andere Ende der Rückzugsfeder (425) gehalten ist, so dass die von der Rückzugsfeder (425) auf das Ladezugseil (420) aufgebrachte Zugkraft entsprechend der Anzahl der Zugrollen (420D) nur einen Bruchteil der Federkraft der Rückzugsfeder (425) entspricht (erster Flaschenzug).

24. Injektionsvorrichtung nach Anspruch 23, **dadurch gekennzeichnet, dass** die Vorschubfeder (424) mit dem Aufnahmerahmen (412) über ein Zugseil (424B) verbunden ist, das über mindestens eine Zugrolle (424D) geführt ist, an deren Achse das andere Ende der Vorschubfeder (424) gehalten ist, so dass die von der Vorschubfeder (424) auf das Zugseil (424B) und damit den Vorschubschlitten (424D) aufgebrachte Zugkraft nur einen Bruchteil der Federkraft der Vorschubfeder (424) entspricht (zweiter Flaschenzug).

25. Injektionsvorrichtung nach Anspruch 1, **dadurch gekennzeichnet, dass** dem Betätigungselement und/ oder dem Injektionsschlitten (440,450) eine Dämpfungseinheit (492) zugeordnet ist.

26. Injektionsvorrichtung nach Anspruch 1, **dadurch gekennzeichnet, dass** weitere Bauteile vorgesehen sind, die nach Beendigung des Injektionsvorgangs eine Zeitverzögerung (TV) bis zum Beginn des Rückholhubs (H3) bewirken.

27. Injektionsvorrichtung nach Anspruch 26, **dadurch gekennzeichnet, dass** die weiteren Bauteile die kraftschlüssige Kopplung zwischen Stössel (450) und Vorschubschlitten (423) bei sich für die Dauer die Zeitverzögerung (TV) weiter bewegendem Vorschubschlitten (423) aufheben.

28. Injektionsvorrichtung nach Anspruch 27, **dadurch gekennzeichnet, dass** die Dauer der Zeitverzögerung (TV) einstellbar ist.

29. Injektionsvorrichtung nach Anspruch 1, **dadurch gekennzeichnet, dass** ein Volumenadapter (410) in den Stössel (450) einsetzbar ist, der den Injektionshub (H2) und damit die Menge eines Medikaments während des Injektionshubes (H2) vorgibt.

30. Injektionsvorrichtung nach Anspruch 13, 20 und 26, **dadurch gekennzeichnet, dass** mindestens zwei in einem Schlitten (414,514) gelagerte Zahnräder eines Zahnradpaares (413,513) zur Erzeugung einer Übersetzung oder Untersetzung zwischen der Linearbewegung des Schlittens (414,514) und des Vorschubschlittens (423) vorgesehen sind, an dem mindestens ein Federelement zur Erzeugung der Hube (H1,H2,H3) und der Zeitverzögerung (TV) angreift.

31. Injektionsvorrichtung nach Anspruch 30, **dadurch gekennzeichnet, dass** der Vorschubschlitten (423) durch einen Zahnriemen (523) gebildet ist.

## Claims

1. An injection device for a syringe, having a syringe body, a cannula with a needle, and a plunger with a plunger rod, and having at least one actuating element (120, 220, 320) for converting the actuating work, to be performed manually by the patient, into a displacement of the syringe body (101, 201, 301) during an insertion stroke (H1) and a return stroke (H3), and into a displacement of the plunger rod during an injection stroke (H2), with a guide device in which the syringe body (101, 201, 301) is mounted, and with a ram (150, 250, 350) which can be displaced against this in order to displace the plunger rod, and where the actuating work, by means of a single, targeted linear movement of the actuating element (120, 220, 320), is converted into the insertion stroke (H1), the injection stroke (H2) and the return stroke (H3) in such a way that the guide device and the ram (150, 250, 350) are acted on jointly by the actuating element in the insertion stroke (H1) and in such a way that only the ram (150, 250, 350) is acted on in the injection stroke (H2), **characterized in that** the guide device includes a displaceable syringe holder (140, 240, 340) in which the syringe (100, 200, 300, 400) is fixed and which is coupled releasably to the ram (150, 250, 350) and is part of an injection carriage, and, in order to perform a return stroke (H3) corresponding in magnitude substantially to the insertion stroke (H1), the actuating element (120, 220, 320, 420) acts on the injection carriage in a positionally and directionally defined manner by means of locking and coupling elements with intercalation of a further carriage (114A, 260, 323, 423).

2. The injection device as claimed in claim 1, **characterized in that** the actuating element is a push rod (120, 220) which is guided parallel to the injection carriage in a housing (110, 210) and by means of which, when it is pushed into the housing (110, 210), the components for producing the return stroke (H3) are also activated.

3. The injection device as claimed in claim 2, **characterized in that** the components for producing the return stroke (H3) include at least one toothed wheel (113) which engages in the injection carriage (140, 150) and in the push rod (120) and which is mounted in a carriage (114A) displaceable in the housing (110), and **in that** the toothed wheel (113) cooperates with a blocking element which blocks the toothed wheel (113), when insertion stroke (H1) and injection stroke (H2) are performed, and which thereafter releases the toothed wheel (113), as a result of which the linear movement of the push rod (120) is converted into the oppositely directed return stroke (H3) of the injection carriage (140, 150).

4. The injection device as claimed in claim 3, **characterized in that** at least two toothed wheels (113A, 113B) for converting the linear movement of the push rod (120) into the return stroke (H3) are provided in the common carriage (114A).

5. The injection device as claimed in claim 3, **characterized in that** the blocking element is a pawl (114) which is linearly displaceable on the carriage (114A) and which, in the blocking position, engages in the teeth of the toothed wheel (113).

6. The injection device as claimed in claim 3, **characterized in that** the blocking element is a pivot lever (114B) which, in the blocking position, engages in the teeth of the push rod (120).

7. The injection device as claimed in claim 2, **characterized in that** the coupling between syringe holder (140) and ram (150) is effected by two slide blocks (145A, 145B) which can be brought into a releasable positive engagement between syringe holder (140) and housing (110), and between syringe holder (140) and ram (150).

8. The injection device as claimed in claim 2, **characterized in that** the coupling between syringe holder (140) and ram (150) is effected by a further toothed wheel (113C) which is likewise held in the carriage (114A) and which is blocked during the insertion stroke (H1).

9. The injection device as claimed in claim 2, **characterized in that** the components for producing the return stroke (H3) include at least one spring element (261A, 261B) as energy accumulator which, before the start of the injection, is pretensioned by the push rod (220) (tensioning stroke) and, after the injection stroke (H2), is released, in order to produce the return stroke (H3) by acting abruptly on a return carriage (260) which is releasably connected to the injection carriage and which bears on the syringe holder (240).

10. The injection device as claimed in claim 2, **characterized in that** a rotatably mounted control lever (221) is provided in the push rod (220), one end of this control lever (221) engaging in the injection carriage (240, 250) when the tensioning stroke has been completed.

11. The injection device as claimed in claim 10, **characterized in that** the control lever (221), by turning about a control angle, also effects the release of the coupling between syringe holder (240) and ram (250) at the transition from the insertion stroke (H1) to the injection stroke (H2).

12. The injection device as claimed in claim 9, **characterized in that** the return carriage (260) has pincer-like locking elements (262A, 262B) which, after the injection stroke (H2), engage in recesses (226A, 226B) of the push rod (220) and release the return stroke (H3).

13. The injection device as claimed in claim 1, **characterized in that** the actuating element includes a pull-out loading bar (320) which, when pulled out from the housing (310), pretensions at least one advancer spring (324) as energy accumulator, and a trigger mechanism (370) which, after activation, releases the injection carriage (340, 350) acted upon by the advancer spring (324) via an advancer carriage (323) for automatic execution of insertion stroke (H1), injection stroke (H2) and return stroke (H3).

14. The injection device as claimed in claim 13, **characterized in that** the pull-out loading bar (320), after it has been pulled out from the housing (310), pretensions at least one restoring spring (325) as energy accumulator for automatic return of the pull-out loading bar (320).

15. The injection device as claimed in claim 13, **characterized in that** the advancer spring (324) and the restoring springs (325) are scroll springs.

16. The injection device as claimed in claim 14, **characterized in that** the trigger mechanism (370) is coupled to at least one safety element (371) which in particular permits triggering only when the injection device is placed on the insertion site.

17. The injection device as claimed in claim 13, **characterized in that** pull-out loading bar (320), advancer springs (324, 325), injection carriage (340, 350) and advancer carriage (323) are held in a receiving frame (312) in such a way that they can be displaced parallel to one another.

18. The injection device as claimed in one of the preceding claims, **characterized in that**, in order to control the processes, in particular the sequence of insertion stroke (H1), injection stroke (H2) and return stroke (H3), control elements that can be brought into and out of positive/frictional engagement with one another are provided, in particular on the actuating element (120, 220, 320), on the syringe holder (140, 240, 340), on the ram (150, 250, 350) and on the housing (110, 210) or receiving frame (312).

19. The injection device as claimed in claim 18, **characterized in that** the control elements include elastic sections, locking cams, slide-on planes and cutouts.

20. The injection device as claimed in claim 13, **characterized in that**, in order to pretension the advancer spring (424), the pull-out loading bar is replaced by a pull-out loading wire (420), one end of which has a grip (420B) on an end of the housing (410), and which has a carrier (420A) which is connected to the advancer spring (424) and engages on the advancer carriage (423) when the grip (420B) is pulled out.

21. The injection device as claimed in claims 14 and 20, **characterized in that** the pretensioning of the restoring spring (425) likewise takes place via the grip (420B) and the pull-out loading wire (420), as a result of which the pull-out loading wire (420) is pulled into the housing (410) until it abuts against the grip (420B) on the housing (410).

22. The injection device as claimed in claims 20 and 21, **characterized in that** advancer spring (424) and restoring spring (425) are designed as helical springs, one end of which is secured in a frame (412) held in the housing (410), and the other end of which is connected to the pull-out loading wire (420) either directly or via the carrier (420A).

23. The injection device as claimed in claim 22, **characterized in that** the other end of the pull-out loading wire (420) is connected to a receiving frame (412) held in the housing and is guided over at least one pull roller (420D) on whose shaft the other end of the restoring spring (425) is held, so that the tensile force applied by the restoring spring (425) on the pull-out loading wire (420) corresponds according to the number of pull rollers (420D) only to a fraction of the spring force of the restoring spring (425) (first pulley block).

24. The injection device as claimed in claim 23, **characterized in that** the advancer spring (424) is connected to the receiving frame (412) via a traction wire (424B) which is guided over at least one pull roller (424D) on whose shaft the other end of the advancer spring (424) is held, so that the tensile force applied by the advancer spring (424) to the traction wire (424B) and thus to the advancer carriage (424D) is only a fraction of the spring force of the advancer spring (424) (second pulley block).

25. The injection device as claimed in claim 1, **characterized in that** a damping unit (492) is assigned to the actuating element and/or to the injection carriage (440, 450).

26. The injection device as claimed in claim 1, **characterized in that** additional components are provided which produce a time delay (TV) between the completion of the injection procedure and the start of the return stroke (H3).

27. The injection device as claimed in claim 26, **characterized in that** the additional components cancel the frictional coupling between ram (450) and advancer carriage (423) as the advancer carriage (423) continues to move for the duration of the time delay (TV).

28. The injection device as claimed in claim 27, **characterized in that** the duration of the time delay (TV) is adjustable.

29. The injection device as claimed in claim 1, **characterized in that** a volume adapter (410) can be inserted into the ram (450) and predetermines the injection stroke (H2) and thus the quantity of a medicament that is administered during the injection stroke (H2).

30. The injection device as claimed in claims 13, 20 and 26, **characterized in that** at least two toothed wheels mounted in a carriage (414, 415) and belonging to a pair of toothed wheels (413, 513) for gearing up or gearing down between the linear movement of the carriage (414, 514) and of the advancer carriage (423) are provided, on which at least one spring element engages for producing the strokes (H1, H2, H3) and the time delay (TV).

31. The injection device as claimed in claim 30, **characterized in that** the advancer carriage (423) is formed by a toothed belt (523).

## Revendications

1. Dispositif d'injection pour une seringue comprenant un corps de seringue, une canule avec aiguille, un piston avec tige de piston, et comprenant au moins un organe d'actionnement (120, 220, 320) pour transformer le travail d'actionnement, à exécuter manuellement par le patient, en un déplacement du corps de seringue (101, 201, 301) pendant une course de piqûre (H1) et une course de retour (H3), ainsi qu'en un déplacement de la tige de piston pendant une course d'injection (H2), comprenant un dispositif de guidage pour maintenir le corps de seringue (101, 201, 301) et un poussoir (150, 250, 350), coulissant par rapport à ce dispositif de guidage pour le déplacement de la tige de piston, dans lequel le travail d'actionnement est transformé, au moyen d'un seul mouvement linéaire dirigé de l'organe d'actionnement (120, 220, 320), en la course de piqûre (H1), la course d'injection (H2) et la course de retour (H3) de telle manière que le dispositif de guidage et le poussoir (150, 250, 350) sont sollicités conjointement par l'organe d'actionnement pour la course de piqûre (H1), et que seul le poussoir (150, 250, 350) est sollicité pour la course d'injection (H2) ;
**caractérisé en ce que** le dispositif de guidage comprend un support de seringue (140, 240, 340) déplaçable, dans lequel est fixée la seringue (100, 200, 300, 400), qui est couplé de manière amovible avec le poussoir (150, 250, 350) et fait partie d'un chariot d'injection, lequel, pour la réalisation d'une course de retour (H3) qui en valeur correspond sensiblement à la course de piqûre (H1), est sollicité par l'organe d'actionnement (120, 220, 320, 420) de façon définie en position et en direction au moyen d'éléments d'enclenchement et de couplage, par l'intermédiaire d'un autre chariot (114A, 260, 323, 423).

2. Dispositif d'injection selon la revendication 1, **caractérisé en ce que** l'organe d'actionnement est une tige de poussée (120, 220) guidée dans un boîtier (110, 210) parallèlement au chariot d'injection, et par l'enfoncement de cette tige de poussée dans le boîtier (110, 210), les composants sont eux aussi actionnés et / ou activés pour produire la course de retour (H3).

3. Dispositif d'injection selon la revendication 2, **caractérisé en ce que** les composants, pour produire la course de retour (H3), comprennent au moins une roue dentée (113) engrenant avec le chariot d'injection (140, 150) et la tige de poussée (120), ladite roue dentée étant montée dans un chariot (114A) déplaçable dans le boîtier (110), et **en ce que** la roue dentée (113) coopère avec un élément de blocage qui bloque la roue dentée (113) lors de l'exécution de la course de piqûre (H1) et de la course d'injection (H2), et qui libère ensuite la roue dentée (113), de sorte que le mouvement linéaire de la tige de poussée (120) est transformé en la course de retour (H3) du chariot d'injection (140, 150), dirigée en sens contraire.

4. Dispositif d'injection selon la revendication 3, **caractérisé en ce qu'**au moins deux roues dentées (113A, 113B) sont prévues dans le chariot commun (114A) pour produire un rapport dans la transmission du mouvement linéaire de la tige de poussée (120) en la course de retour (H3).

5. Dispositif d'injection selon la revendication 3, **caractérisé en ce que** l'élément de blocage est un cliquet (114) linéairement déplaçable sur le chariot (114A), lequel cliquet s'engage dans les dents de la roue dentée (113) dans la position de blocage.

6. Dispositif d'injection selon la revendication 3, **caractérisé en ce que** l'élément de blocage est un levier basculant (114B), qui dans la position de blocage s'engage dans la denture de la tige de poussée (120).

7. Dispositif d'injection selon la revendication 2, **caractérisé en ce que** l'accouplement entre le support de seringue (140) et le poussoir (150) est effectué par deux blocs coulissants (145A, 145B), qui peuvent être amenés dans une liaison de verrouillage amovible entre le support de seringue (140) et le boîtier (110), et entre le support de seringue (140) et le poussoir (150).

8. Dispositif d'injection selon la revendication 2, **caractérisé en ce que** l'accouplement entre le support de seringue (140) et le poussoir (150) est effectué par une autre roue dentée (113C), également montée dans le chariot (114A), la roue dentée étant bloquée pendant la course de piqûre (H1).

9. Dispositif d'injection selon la revendication 2, **caractérisé en ce que** les composants pour produire la course de retour (H3) comprennent au moins un élément à ressort (261A, 261B) comme accumulateur d'énergie, qui avant le début de l'injection est précontraint par la tige de poussée (220) (course de tension), et qui est libéré après la course d'injection (H2) pour la sollicitation impulsionnelle d'un chariot de retour (260) relié de façon amovible au chariot d'injection et adjacent au support de seringue (240), pour produire la course de retour (H3).

10. Dispositif d'injection selon la revendication 2, **caractérisé en ce que** dans la tige de poussée (220) est prévu un levier de commande (221) monté de manière rotative, dont une extrémité s'engage dans le chariot d'injection (240, 250) lorsque la course de tension est terminée.

11. Dispositif d'injection selon la revendication 10, **caractérisé en ce que** le levier de commande (221) provoque également, par rotation selon un angle de commande, la séparation de l'accouplement entre le support de seringue (240) et le poussoir (250) au passage de la course de piqûre (H1) à la course d'injection (H2).

12. Dispositif d'injection selon la revendication 9, **caractérisé en ce que** le chariot de retour (260) comprend des éléments d'enclenchement (262A, 262B) en forme de pince, qui après la course d'injection (H2) s'enfoncent dans des évidements (226A, 226B) de la tige de poussée (220) et libèrent la course de retour (H3).

13. Dispositif d'injection selon la revendication 1, **caractérisé en ce que** l'organe d'actionnement comprend une tige d'armement (320) fonctionnant en traction, qui lorsqu'elle est tirée vers l'extérieur du boîtier (310) précontraint au moins un ressort de propulsion (324) comme accumulateur d'énergie, ainsi qu'un mécanisme de déclenchement (370), qui après activation libère le chariot d'injection (340, 350) sollicité par le ressort de propulsion (324) par l'intermédiaire d'un chariot de propulsion (323) pour l'exécution automatique de la course de piqûre (H1), la course d'injection (H2) et la course de retour (H3).

14. Dispositif d'injection selon la revendication 13, **caractérisé en ce qu'**une fois tirée hors du boîtier (310) la tige d'armement (320) fonctionnant en traction précontraint en tant qu'accumulateur d'énergie au moins un ressort de rappel (325) pour le retour automatique de la tige d'armement (320) fonctionnant en traction.

15. Dispositif d'injection selon la revendication 13, **caractérisé en ce que** le ressort de propulsion (324) et les ressorts de rappel (325) sont des ressorts à enroulement.

16. Dispositif d'injection selon la revendication 14, **caractérisé en ce que** le mécanisme de déclenchement (370) est couplé avec au moins un élément de sécurité (371), qui en particulier permet le déclenchement seulement lorsque le dispositif d'injection est placé sur le point de piqûre.

17. Dispositif d'injection selon la revendication 13, **caractérisé en ce que** la tige d'armement (320) fonctionnant en traction, les ressorts de propulsion (324, 325), le chariot d'injection (340, 350) et le chariot de propulsion (323) sont montés déplaçable parallèlement entre eux dans un châssis de montage (312).

18. Dispositif d'injection selon l'une quelconque des revendications précédentes, **caractérisé en ce que**, pour la commande des processus, en particulier la succession de la course de piqûre (H1), la course d'injection (H2) et la course de retour (H3), sont prévus des éléments de commande, qui peuvent être mis dans ou libérés d'une liaison par serrage ou par formes conjuguées les uns avec les autres, en particulier sur l'organe d'actionnement (120, 220, 320), sur le support de seringue (140, 240, 340), sur le poussoir (150, 250, 350) et sur le boîtier (110, 210) ou le châssis de montage (312).

19. Dispositif d'injection selon la revendication 18, **caractérisé en ce que** les éléments de commande comprennent des parties élastiques, des saillies d'enclenchement, des plans de glissement, et des évidements.

20. Dispositif d'injection selon la revendication 13, **caractérisé en ce que** pour pré-contraindre le ressort de propulsion (424), la tige d'armement fonctionnant en traction est remplacée par un câble d'armement (420) fonctionnant en traction dont une extrémité comporte une poignée (420B) à une extrémité du boîtier (410), qui comprend un taquet (420A) qui est relié au ressort de propulsion, et vient en prise avec le chariot de propulsion (423) lorsque l'on tire la poignée (420B).

21. Dispositif d'injection selon les revendications 14 et 20, **caractérisé en ce que** la précontrainte du ressort de rappel (425) s'effectue également par la poignée (420B) et le câble d'armement (420) fonctionnant en traction, ledit câble d'armement (420) fonctionnant en traction étant de ce fait rétracté dans le boîtier (410) jusqu'à butée de la poignée (420B) sur le boîtier (410).

22. Dispositif d'injection selon les revendications 20 et 21, **caractérisé en ce que** le ressort de propulsion (424) et le ressort de rappel (425) sont formés par des ressorts spirale, dont une extrémité est fixée dans un châssis (412) maintenu dans le boîtier (410), et dont l'autre extrémité est reliée directement ou par l'intermédiaire du taquet (420A) avec le câble d'armement (420) fonctionnant en traction.

23. Dispositif d'injection selon la revendication 22, **caractérisé en ce que** l'autre extrémité du câble d'armement (420) fonctionnant en traction est reliée à un châssis de montage (412) maintenu dans le boîtier et est guidée par l'intermédiaire d'au moins un galet tendeur (420D) dont l'axe supporte l'autre extrémité du ressort de rappel, de sorte que la force de traction appliquée par le ressort de rappel (425) sur le câble d'armement (420) fonctionnant en traction ne corresponde qu'à une fraction de la force élastique du ressort de rappel (425), selon le nombre des galets tendeurs (420D) (première chape de poulie).

24. Dispositif d'injection selon la revendication 23, **caractérisé en ce que** le ressort de propulsion (424) est relié au châssis de montage (412) par l'intermédiaire d'un câble de traction (424B) qui est guidé par au moins un galet tendeur (424D), dont l'axe supporte l'autre extrémité du ressort de propulsion (424), de sorte que la force de traction appliquée par le ressort de propulsion (424) sur le câble de traction (424B) et donc le chariot de propulsion (424D) corresponde seulement à une fraction de la force élastique du ressort de propulsion (deuxième chape de poulie).

25. Dispositif d'injection selon la revendication 1, **caractérisé en ce qu'**une unité d'amortissement (492) est associée à l'organe d'actionnement et / ou au chariot d'injection (440, 450).

26. Dispositif d'injection selon la revendication 1, **caractérisé en ce que** d'autres composants sont prévus, qui provoquent une temporisation (TV) après la fin du processus d'injection jusqu'au début de la course de retour (H3).

27. Dispositif d'injection selon la revendication 26, **caractérisé en ce que** les autres composants suppriment l'accouplement par adhérence entre le poussoir (450) et le chariot de propulsion (423) pendant que le chariot de propulsion (423) continue son mouvement sur la durée de la temporisation (TV).

28. Dispositif d'injection selon la revendication 27, **caractérisé en ce que** la durée de la temporisation (TV) est réglable.

29. Dispositif d'injection selon la revendication 1, **caractérisé en ce qu'**on peut installer dans le poussoir (450) un adaptateur de volume (410) qui définit la course d'injection (H2) et donc la quantité d'un médicament pendant la course d'injection (H2).

30. Dispositif d'injection selon les revendications 13, 20 et 26, **caractérisé en ce qu'**au moins deux roues dentées d'une paire d'engrenage (413, 513), montées dans un chariot (414, 514) sont prévues afin de produire un rapport de surmultiplication ou de réduction de transmission entre le mouvement linéaire du chariot (414, 514) et le chariot de propulsion (423), sur lequel agit au moins un élément à ressort pour produire les courses (H1, H2, H3) et la temporisation (TV).

31. Dispositif d'injection selon la revendication 30, **caractérisé en ce que** le chariot de propulsion (423) est formé par une courroie dentée (523).
